# EUROPEAN PATENT APPLICATION

(11) **EP 1 054 059 A1**
(43) Date of publication of application: **22.11.2000**
(21) Application number: 99201543.8
(22) Date of filing: 17.05.1999
(51) Int. Cl.: C12N 15/12, C12N 5/10, C12N 1/21, C12N 1/15, C07K 14/47, C07K 16/18, A01K 67/027, G01N 33/50, G01N 33/53, G01N 33/68, C12Q 1/68, A61K 31/70, A61K 49/00, A61K 38/17, A61K 39/395

(54) **Novel cDNAs encoding catenin-binding proteins with function in signalling and/or gene regulation**

(71) Applicant: Vlaams Interuniversitair Instituut voor Biotechnologie vzw., 9052 Zwijnaarde (BE)
(72) Inventor: Van Roy, Frans, B-9070 Destelbergen (BE); Vanlandschoot, Ann, B-9570 Deftinge (BE); Janssens, Barbara, B-9000 Gent (BE)

(57) **Abstract**

The invention relates to the field of drug discovery, diagnosis, prognosis and treatment of cancer and neurological disorders. The invention provides among others access to and insight in protein-protein or protein-DNA interactions in a signal transduction or transcriptional pathway controlling cell growth or development throughout a wide range of cells and tissues of the body, and provides means, such as nucleic acid, protein, cells and experimental animals and methods to identify candidate drugs, for example for use in therapy of cancer or neurological disorders. As an example of a catenin-binding protein with function in intracellular signalling or gene regulation, the invention provides an isolated and/or recombinant nucleic acid or a functional fragment, homologue or derivative thereof, corresponding to a zinc finger gene with a nucleic acid sequence as shown in figure 1 and encoding a zinc finger protein, or fragment thereof, capable of complexing with a neurally expressed catenin.

## Description

The invention relates to the field of drug discovery, diagnosis and treatment of cancer and neurological disorders.

Despite extensive knowledge relating to the multitude of cancer forms (varying in appearance from solid tumours and related metastases in distinct parts of the body to leukaemia's of blood cells that circulate throughout the body, and varying from being totally benign to being aggressively malignant) effective therapy of cancer is difficult and in general restricted to three types; treatment via radiation, via chemotherapy and via surgery. Possibilities for a more specific therapy, directed against the underlying cause of a specific cancer or groups of cancers are currently virtually non-existing. Extensive efforts are directed at providing such specific drugs through drug discovery attempts that try to identify candidate drugs for specific therapy of cancer or neurological disorders.

Development of cancer often starts with changes in a cell that lead to the unrestricted development and division of that first cell into an ever dividing population of cells. These changes are often an accumulation of mutations or other alterations in key genes that occur chronologically, whereby the mutated cell population looses its original, often specialised character and acquires more and more of a cancerous nature. Normal processes of growth regulation of cells are dysfunctioning in the altered cells. Transcription of genes that are normally only little expressed in said cell type is possibly no longer controlled in cancerous cells.

Activation of transcription of genes by transcription factors that would otherwise be dormant in the specific cell type can for example lead to the so typical unrestricted growth and neoplastic nature of cancer. Examples are mutations in suppressor genes that function normally by generating proteins that are suppressing transcriptional pathways which are no longer of use in a specialised cell. Mutated suppressor genes do not longer help keep the growth of a cell under control. Drugs directed against or intervening with the specific protein-protein or protein-DNA interactions in transcriptional and/or signalling pathways controlling cell growth, cell differentiation or development can be considered typical candidate drugs for later use in specific therapy of cancer or neurological disorders, especially when such pathways have gone awry and lead to unrestricted growth or aberrant differentiation of cells. In the case of αN-catenin, which is mainly neurally expressed, a broader or additive interpretation in relationship to neural dysfunctions is obvious.

The cadherin superfamily represents several cadherins with function in cell-cell adhesion, morphogenesis and tissue homeostasis (Takeichi, 1991; Kemler, 1992; Suzuki, 1996). The transmembrane glycoprotein E-cadherin is the best-studied prototype of this family and has been identified as a potent suppressor of invasion (Behrens et al., 1989; Frixen et al., 1991; Vleminckx et al., 1991). Recent studies revealed proof for a tumor suppressor role of human E-cadherin as the encoding gene behaves according to the two-hit model of Knudson (1985) in infiltrative lobular cancers (Berx et al. 1995 and 1996) and diffuse gastric cancers (Becker et al., 1994 and 1996).

Cadherins function as cell-cell adhesion molecules by homophilic interactions with other cadherin molecules, but linkage to the actin cytoskeleton is also essential. The latter is achieved by the catenins (catena means chain) (Ozawa et al., 1990; Cowin, 1994), which comprise the Armadillo proteins (e.g. β-catenin, plakoglobin and p120^{ctn}) and the vinculin-like α-catenins. The Armadillo catenins are proteins, known to be associated with the cytoplasmic domain of cadherins. In turn, the α-catenins link β-catenin and plakoglobin to the actin cytoskeleton.

These catenins were also found to be associated with the cytoplasmic tumor suppressor gene product APC (adenomatous polyposis coli) (Peifer, 1993; Su et al., 1993). A typical example of a signal transduction pathway (via β-catenin) gone wrong and leading to development of cancer, can be found with APC. The APC protein is linked to the microtubular cytoskeleton. Moreover, in the desmosomes, plakoglobin mediates a link between desmosomal cadherins and the cytokeratin cytoskeleton via desmoplakin (Korman et al., 1989; Kowalczyk et al., 1997).

For α-catenin, two subtypes have been identified, αE-catenin (epithelial form) (Nagafuchi et al., 1991; Herrenknecht et al., 1991) and αN-catenin (neural form) (Hirano et al., 1992). Moreover, for both subtypes two isoforms, resulting from alternative splice events, were identified (Oda et al., 1993; Uchida et al., 1994; Rimm et al., 1994). A tissue specific distribution for either of the subtypes has been reported. The epithelial αE-catenin is expressed in a wide variety of tissues, but only low levels of expression were observed in the central nervous system (CNS) (Nagafuchi et al., 1991). In contrast, αN-catenin expression is more restricted to particular tissues including the nervous system in which it is generally expressed (Hirano et al., 1992; Uchida et al., 1994).

One of the most intriguing discoveries in this field is the recently described association of LEF-1 (lymphocyte enhancer-binding factor-1), an architectural transcription factor (Love et al., 1995) with β-catenin (Behrens et al., 1996). The interaction between β-catenin and LEF-1 leads to nuclear translocation of these two proteins, implicating a central role for β-catenin in the transcriptional regulation of target genes, which can lead to tumorigeneity (Huber et al., 1996; Peifer, 1997). Among the target genes induced by the β-catenin/LEF-1 complex are the myc proto-oncogene (He et al., 1998) and the cyclin D1 gene (Tetsu and McCormick, 1999).

Cadherin-catenin-cytoskeleton complexes are key elements of cell-cell adhesion and regulation of motility, but it may be clear that the importance of nuclear signalling by catenins is gaining interest and may be critical in tumorigeneity, invasion and metastasis. However, despite all the knowledge of the cadherins and catenins it was not known what proteins are capable of translocating catenins to the nucleus, and how catenins might exert their effect on intracellular signalling and on the transcription of genes in the cell. Only with the means and methods of the current invention a key step has become apparent. Moreover, through the identification of a key step in the translocation of catenins to the nucleus it has now become possible to develop means and methods interfering with said process in for instance tumorigeneity, invasion and metastasis of cells.

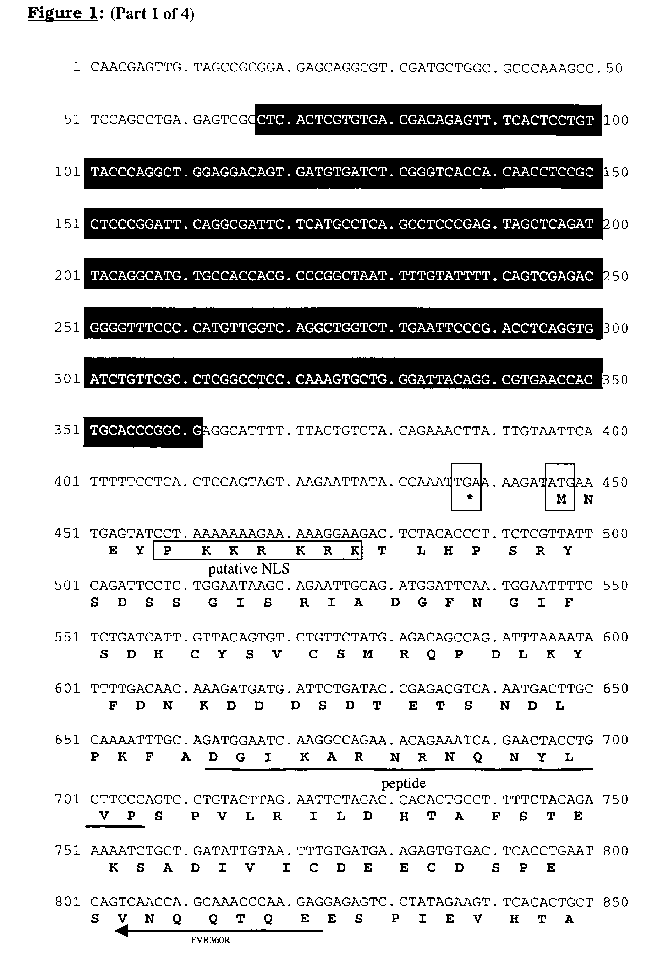

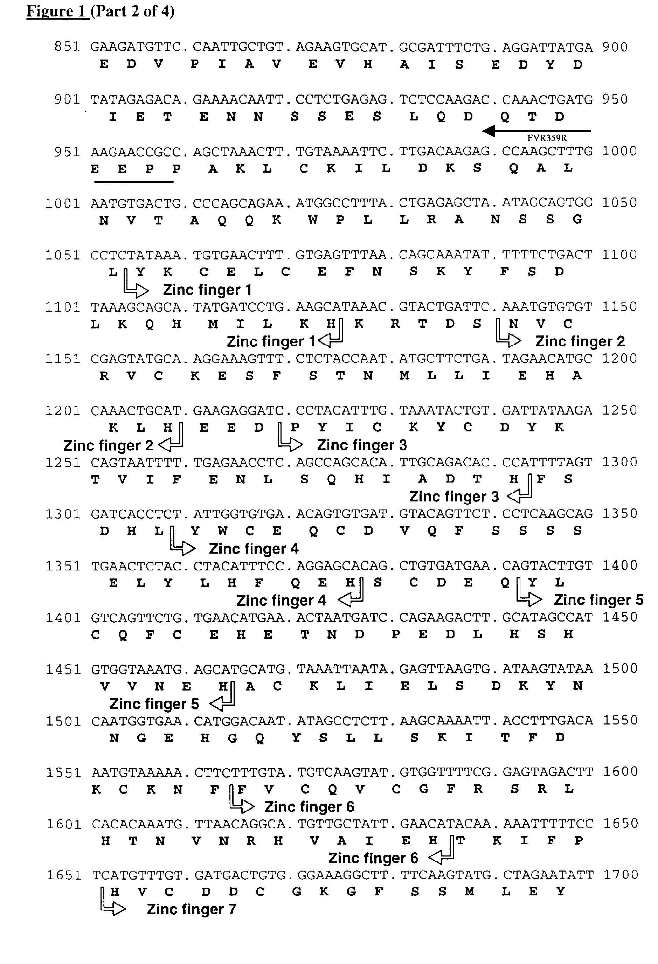

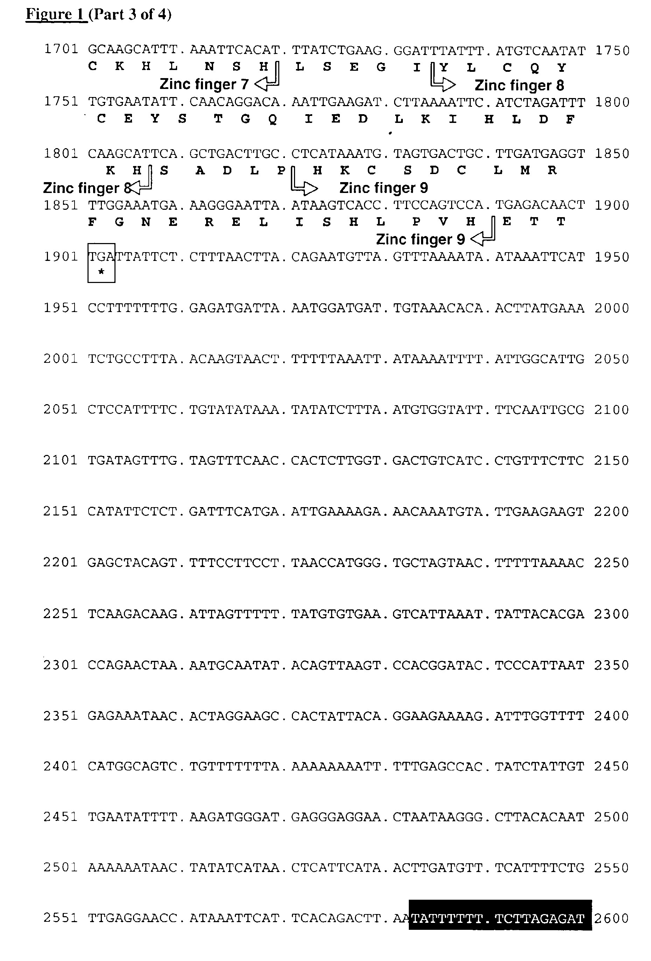

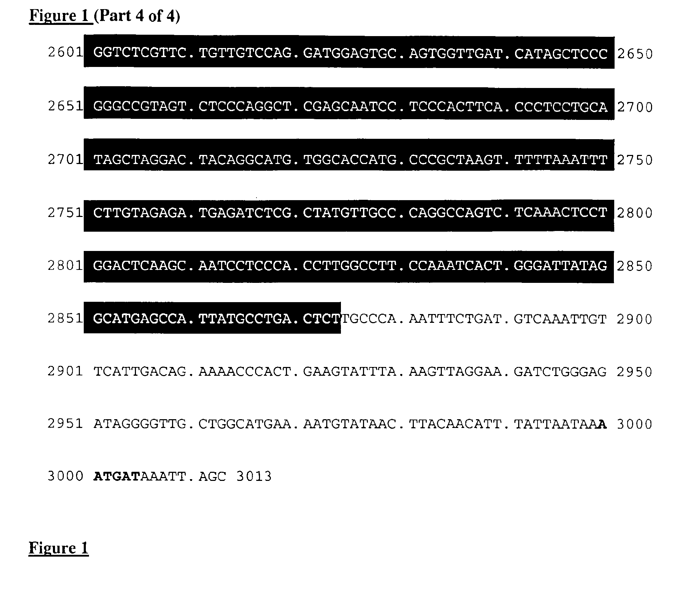

The invention therefore provides among others access to and insight in protein-protein or protein-DNA interactions in a transcriptional pathway controlling cell growth or development throughout a wide range of cells and tissues of the body, and provides means, such as nucleic acid, protein, cells and experimental animals and methods to identify candidate drugs, for example for use in therapy of cancer and/or neurological disorders. Where in this application the definition "nucleic acid" is used, both RNA and DNA, in single or double-stranded fashion, and nucleic acid hybridising thereto is meant. "Homologue" herein meaning a related nucleic acid that can be found with another species. "Derivative" herein meaning a nucleic acid that has been derived by genetic modifications, such as deletions, insertions, and mutations from a distinct nucleic acid or fragment thereof. "Corresponding" herein meaning having a nucleic acid sequence homology of at least 80%, more preferably of at least 90%. The sequence similarities, obtained by the BLAST algorithm (Altschul et al., 1990) are given by P-scores (the more negative, the higher the similarity), not by percentages. Nevertheless, nucleotide sequence homology can be expressed as percentages (numbers of identical nucleotides per 100 nucleotides).
The invention provides an isolated and/or recombinant nucleic acid or a functional fragment, homologue or derivative thereof, corresponding to a catenin-binding protein with function in signal transduction or gene regulatory pathways more specifically to an isolated and/or recombinant nucleic acid or a functional fragment, homologue or derivative thereof, corresponding to for example a zinc finger gene with a nucleic acid sequence as shown in figure 1 and encoding a zinc finger protein, or fragment thereof, said protein capable of complexing or interacting with catenin or fragments thereof. "Functional fragment" herein meaning a nucleic acid or part thereof that is functionally or structurally related to or hybridising with a distinct nucleic acid or fragment thereof. Typical examples of such a functional fragment as provided by the invention are DNA binding elements and/or subcellular localisation signals. For example, further characterisation of nucleic acid according to the invention revealed the presence of nucleic acid encoding protein fragments encoding Cys₂His₂ zinc fingers with DNA binding properties. In addition, in yet another functional fragment, a nuclear localisation signal (NLS, such as PKKRKRK) is found.

The invention also provides a nucleic acid according to the invention wherein said protein is capable of nuclear translocation of αN-catenin but not αE-catenin. Co-expression of a zinc finger protein as provided by the invention or a functional fragment thereof with particular catenins such as αN-catenin leads to a translocation of this catenin into the nucleus. A zinc finger protein as provided by the invention protein can for example be isolated in a two-hybrid screening, using human αN-catenin or another catenin as a bait, and is herein also called a catenin-binding protein.
Up to now, there has been no report of zinc finger proteins binding to catenin or αN-catenin. Furthermore, the invention provides a nucleic acid or derivative thereof corresponding to a nucleic acid or functional fragment thereof encoding a zinc finger protein or other signalling or gene regulatory protein capable of interacting or binding with a catenin, for example an Armadillo catenin or an α-catenin. For example, the invention provides a zinc finger protein encoded by said nucleic acid interacting with catenin, for example with αN-catenin. The catenin protein, forming a complex together with the zinc finger protein, is involved in signalling and gene regulation in for example transcriptional pathways.

In a preferred embodiment, the invention provides nucleic acid according to the invention which is a cDNA molecule, as for example described in the experimental part of this description.

Furthermore, the invention provides an expression vector comprising a nucleic acid according to the invention. An example of such a vector can be found in the experimental part of the description. It is within the skills of the artisan to provide vectors that have been provided with single or multiple nucleic acid changes, deletions and/or insertions or other mutations of a nucleic acid sequence.

Furthermore, the invention provides a cell capable of expressing the zinc finger protein or functional fragments thereof as provided by the invention. For example, the invention provides a cell comprising a genome in which a nucleic acid sequence corresponding to a nucleic acid according to the invention has been modified. Such a modification can comprise a (for example site-directed or transposon-directed or chemically induced) mutation of a nucleic acid encoding a (fragment) of a gene encoding an catenin-binding zinc finger protein, be it in the intronic or exonic sequences of said gene.

The invention also provides a cell comprising a nucleic acid according to the invention that has been introduced via recombinant means known to the skilled artisan, for example via homologous recombination techniques or by using a vector according to the invention. An example of such a cell according to the invention is provided in the experimental part of the description wherein said cell is a yeast cell, such as for use in a two-hybrid interaction assay.

The invention also provides a cell capable of expressing a catenin-binding protein or derivative or fragment thereof, said protein comprising a catenin binding domain or capable of complexing with catenin. Such a cell provided by the invention is for example derived of a cell comprising a genome in which a nucleic acid sequence corresponding to a nucleic acid according to the invention has been modified or derived of a cell comprising a nucleic acid according to the invention. Expression of proteins in recombinant cells is in itself a technique available to the average artisan. An example is provided in the experimental part wherein yeast cells expressing a catenin-binding protein are provided.

Furthermore, the invention provides an animal comprising a cell according to the invention. Such an animal is for example a transgenic animal obtained by modifying an embryonic stem cell. Stem cell modifications, as well as modifications of other cells, are known to the average skilled artisan. Such an animal is for example a homozygous knock-out animal or a heterozygous animal, for example a cross between a knock-out animal with a wild type animal or is otherwise modified in a nucleic acid fragment in its genome that is corresponding to a nucleic acid provided by the invention. A preferred embodiment of the invention is wherein said animal is a mouse.

The invention also provides a zinc finger protein or derivative or fragment thereof, said protein capable of complexing with catenin, preferably with αN-catenin. Preferable, such a protein is derived by recombinant techniques and encoded by a nucleic acid according to the invention. Proteins, be it natural proteins or recombinant versions thereof can easily be isolated by a skilled artisan, for example when at least a part of the amino acid sequence, or a specific antibody directed against the protein, is provided. The invention provides such an isolated or recombinant catenin-binding zinc finger protein or (poly)peptide or derivatives or fragments thereof. In addition, the invention provides an antibody specifically directed against a such protein or (poly)peptide or derivative or fragment thereof according the invention. It is within the skills of the average skilled artisan to provide a (synthetic) antibody directed against a protein or fragment thereof, for example once the amino acid sequence or the isolated protein is provided.

The invention also provides a method for identifying a candidate drug comprising use of a cell or an animal or a protein or an antibody according to the invention. Candidate drugs, often first selected or generated via combinatorial chemistry, can now be tested and identified using a method provided by the invention. Such a candidate drug or compound can for example be tested on and selected for their effect on zinc finger protein regulated nuclear translocation of catenin as for example measured according to the experimental part of the description. For example, a test compound or drug which inhibits the nuclear translocation of catenin is a candidate drug for therapy of cancer or neurological disorders. The mRNA encoding the zinc finger protein is found in all human tissue examined so far, with a low expression level in most of the tissues and a stronger mRNA signal in pituitary gland and adrenal gland. Lung, placenta, fetal liver and fetal lung also showed expression of the mRNA, but lower than the pituitary gland and the adrenal gland (see section Multiple tissue RNA dot blot). The partial cDNA encoding the zinc finger protein was isolated from a human kidney cDNA library and the 5'RACE fragment was obtained using mammary gland mRNA. On the other hand, αN-catenin is known to be mainly neurally expressed. Furthermore, the invention provides a method for diagnosing cancer or neurological disorders comprising use of a nucleic acid, a cell or an animal, or a protein or an antibody according to the invention. A test or compound which inhibits either interaction of the zinc finger protein with αN-catenin or the interaction of the zinc finger protein with DNA-target sequences, is also considered as a candidate drug for therapy of cancer or nueurological disorders. Other applications are simplified by discrimination between different types of cancer or neurological disorders, aiming at for instance improved diagnosis, prognosis and therapy.

The invention is further explained in the experimental part without limiting the invention.

### Experimental part

Here, we report the isolation, in a yeast two-hybrid screen, of a novel human zinc finger protein associating with a human isoform of α-catenin, termed αN-catenin. The interaction we observed is αN-catenin specific, since the epithelial αE-catenin does not interact with the zinc finger protein using the two-hybrid system. The cDNA of the isolated Cys₂His₂ -type zinc finger protein was completed using 5' RACE technology. In human cells transfected with the former cDNA, the full length zinc finger protein localizes to the nucleus. In human cells transfected with a cDNA of αN-catenin, this protein is expressed in the cytoplasm. When these two cDNAs are co-expressed, both proteins colocalize in the nucleus. These results indicate the formation of a complex between the zinc finger protein and αN-catenin and a subsequent translocation to the nucleus. To date, there has been no report of a nuclear zinc finger protein binding to αN-catenin, nor of nuclear localization of the latter. The presence of the zinc finger domain at the carboxy-terminus, consisting of 9 Cys₂His₂ zinc fingers with putative DNA binding properties, shows that the isolated zinc finger protein might bind to specific DNA sequences. Taking together with the knowledge that αN-catenin is expressed mainly in the neural system, it might play a role in the transcriptional regulation of target genes, in particular in tumors and in the neural system.

### MATERIALS AND METHODS

### Bacterial strains and cell lines

Escherichia coli DH5α (supE44, hsdR17, deoR, recA1, endA1, lacZDM15) and E. coli HB101 (supE44, mcrB, mrr, hsdS20, recA1) were used for transformations, plasmid propagation and isolation. The bacteria were grown in LB medium supplemented with 100 µg/ml ampicillin. For selection of the two-hybrid cDNA-library plasmid, transformed HB101 bacteria were grown on minimal M9 medium, supplemented with 50 µg/ml ampicillin, 40 µg/ml proline, 1 mM thiamine-HCl and 1% of the appropriate amino acid drop out solution. After selection the bacteria were maintained in LB medium supplemented with 50 µg/ml ampicillin.

The HEK293T, a human embryonic kidney cell line transfected with SV40 large T-antigen (SV40 tsA1609) (Graham et al., 1977; DuBridge et al., 1987) was kindly provided by Dr. M. Hall (University of Birmingham, UK), and was used for transient eukaryotic expression. HEK293T cells were grown at 37°C with 5% CO₂ in Dulbecco minimal essential medium (DMEM, Gibco BRL Life Technologies, Paisly, UK) supplemented with 10% FCS, 0.03% glutamine, 100 U/ml penicillin, 100 mg/l streptomycin and 0.4 mM sodium pyruvate.

GLC34 and GLC8 are cell lines derived from small cell lung carcinomas, established as described by de Leij et al. (1985), and kindley made available by Dr. Charles Buys and Dr. Lou de Leij (University of Groningen, the Netherlands). MKN45 is a gastric carcinoma cell line (Motoyama and Watanabe, 1983).

### Plasmids and gene assembly

Restriction enzymes were purchased from Gibco BRL Life Technologies (Paisley, UK) or from New England Biolabs (Beverly, MA, USA). Restriction enzymes were used according to manufacturers' recommendations. All PCR reactions were performed using Vent™ (Biolabs) DNA polymerase. The primers for PCR amplification were either home made (University of Ghent) or obtained from Gibco BRL. The standard PCR mixture, in a reaction volume of 100 µl, contained template cDNA (plasmid), 25 pmol of both specific primers, 200 µM dXTPs and the PCR buffer supplied with Vent™ DNA polymerase. Unless otherwise stated, no additional MgSO₄ was added. Vent™ DNA polymerase was used at 1 U/reaction. The DNA amplification was performed in the PTC-200 Peltier Thermal Cycler PCR System (MJ Research, Watertown, MA). The PCR program started with a DNA denaturating step at 94°C for 3 min, followed by 80°C for 1 min. Cycling conditions were 94°C for 1 min, 50-60°C for 30 sec and 72°C for 2 min. This was repeated for a total of 35 cycles and was followed by a final extension step at 72°C for 10 min.

### Construction of the plasmid encoding the αN-catenin GAL4-DBD hybrid protein for two-hybrid screening

For the two-hybrid screen the almost full-length cDNA of human αN-catenin (residues 4-906) was fused in frame to the GAL4 DNA binding domain in the pAS2 vector (Matchmaker™, Clontech, Palo Alto, CA). This construct was called pAS2ANCTN and was created using amplification by polymerase chain reaction (PCR) and restriction fragments. For construction of this pAS2ANCTN two-hybrid bait plasmid, we used the expression plasmid pPNhANCTN, which contains the human αN-catenin coding sequence flanked by part of the 5' and 3'UTR. The cDNA for αN-catenin was kindly provided by Dr. C. Petit (Institut Pasteur, Paris; Claverie et al., 1993). Using a specific sense primer completed with the XmaI restriction site (underlined) 5'-ACCCCCCGGGGGCAACTTCACCTATCATTC-3' (= FVR137F; Table 4) and a compatible antisense primer 5'-GCCGCCGCCTTCCTTTTCATTTCCGCTCTT-3' (= FVR138R; Table 4), we amplified a PCR fragment from the pPNhANCTN plasmid containing the XmaI restriction site at the 5' end. This PCR fragment was digested with XmaI and BanI and ligated together with a BanI-HindIII fragment of the αN-catenin cDNA in the XmaI-HindIII digested pAS2 vector. The in-frame cloning was confirmed by DNA sequence analysis using the vector specific forward primer 5'-ATCATCGGAAGAGAGTAGTA-3' (= FVR175F; Table 2). To check for the insertion of the complete fragment, the constructed plasmid was also sequenced with a vector specific reverse primer 5'-AAAATCATAAATCATAAGAA-3' (= FVR217R; Table 2). The plasmid was assayed for expression of the fusion protein in yeast using Western blot analysis with an antibody directed against the GAL4 DNA binding domain (anti GAL4 DBD rabbit polyclonal antiserum, UBI, Lake Placid, NY).

### Construction of the plasmids encoding fragments of αN-Catenin fused to the GAL4-DBD

Several restriction fragments of pAS2ANCTN were subcloned into the pGBT9 (Clontech, Palo Alto, CA; Bartel et al., 1993) to construct plasmids encoding different parts of the αN-catenin protein fused to the GAL4 DNA binding domain. An EcoRI-BamHI fragment (encoding residues 4-193), an EcoRI-PstI fragment (encoding residues 4-535), a PstI fragment (encoding residues 535-787) and a XhoI-SalI fragment (encoding residues 543-906) were isolated and ligated in frame with the GAL4 DNA binding domain in the pGBT9 vector digested with the appropriate restriction enzymes. The plasmids were designated pGBT9ANCTN(EcoRI-BamHI), pGBT9ANCTN(EcoRI-PstI), pGBT9ANCTN(PstI1618-2375) and pGBT9ANCTN-(XhoI-SalI), respectively. The in-frame cloning of the fragments was confirmed by DNA sequence analysis using the vector specific forward primer 5'-ATCATCGGAAGAGAGTAGTA-3' (= FVR175F; Table 2). The inserts were also sequenced with the vector-specific reverse primer (= FVR217R; Table 2) 5'-AAAATCATA-AATCATAAGAA-3'.

### Construction of the plasmid encoding αE-Catenin fused to the GAL4-DBD

A human fetal kidney 5' Stretch cDNA library (Clontech, Palo Alto, CA) in vector λDR2 was screened with a ³²P-labeled αE-catenin-specific probe. This resulted in the isolation of the pDR2αECTN plasmid (phages were converted in vivo into the pDR2-derived plasmids according to the manufacturers protocol), containing the full-length cDNA of αE-catenin. The plasmid was digested with SalI, SphI and Eco47III. The SalI/SphI and the Eco47II/SphI fragments were ligated into the SmaI/SalI digested pGBT9 vector. The two-hybrid plasmid was called pGBT9αECTN and was analyzed for in-frame cloning by DNA sequence analysis using the vector-specific forward primer FVR175F (Table 2). The insertion of the fragments was confirmed by sequencing the 3' end using the vector-specific primer FVR217R (Table 2).

### Construction of the plasmids encoding the amino-terminal part of ANC_2H01 fused to the GAL4-AD

The ANC_2H01 clone, isolated from the human kidney cDNA library by performing a two-hybrid screen with αN-catenin as a bait, was digested with BamHI. The 700-bp fragment was isolated and subcloned into the BamHI digested pGAD10 vector (Clontech, Palo Alto, CA). The cDNA insert encodes for the amino-terminal part plus two of the nine zinc fingers. The in-frame cloning was confirmed by DNA sequence analysis. We designated the plasmid ANC_2H01/BamHI.

Using amplification by PCR with compatible primers containing an additional restriction site, we subcloned parts of the ANC_2H01 cDNA encoding for the amino-terminal part of the protein, lacking any functional zinc finger. For this construct, PCR amplification from the initial ANC_2H01 two-hybrid clone was performed using the forward primer 5'-GCACTATGGCCAGAAACAGAAATCAGA-3' (= FVR345F; Table 1), compatible with the reverse primer 5'-GGAATTCTGGGCAGTCACATTCAAAG-3' (= FVR346R; Table 1), which included an EcoRI restriction site (underlined). The amplified fragment was digested with XbaI/EcoRI. A second fragment was isolated as a BamHI/XbaI fragment of ANC_2H01. Both fragments were ligated in the two-hybrid vector pGAD10 double digested by BamHI/EcoRI. We named the final plasmid ANC_2H01/500.

Another construct, containing a cDNA fragment encoding the amino-terminal part of the initial ANC_2H01 plus half of the first zinc finger, was made using the same strategy. We amplified a PCR fragment from ANC_2H01 using the same forward primer (= FVR345F; Table 1) that was compatible with the reverse primer 5'-GGAATTCCATATGCTGCTTTAAGTCAG-3' (= FVR347R; Table 1), in which an EcoRI site (underlined) was included. The plasmid was called ANC_2H01/600. The in-frame cloning and the insertion of the fragments were confirmed by DNA sequence analysis, using primers FVR174F (Table 2) and FVR192R (Table 2) for each of the constructed plasmids.

### Construction of the plasmid for eukaryotic expression of epitope tagged protein.

We constructed an expression vector for eukaryotic expression of the ANC_2H01 full-length protein fused to the E-tag. We used the NotI-KpnI fragment of the vector pEFHOBES (a kind gift from M. Van de Craen, Department of Molecular Biology, VIB-University of Ghent, Belgium), consisting of the expression vector pEF-BOS (Mizushima and Nagata, 1990), in which the E-tag derived from plasmid pCANTAB5E (Pharmacia), is inserted. To construct the expression vector pEFBOSANC_2H01E, which encodes for the ANC_2H01 protein fused at its amino-terminal side to the E-tag, a three points ligation was set up. The first fragment was amplified from the cloned 5' RACE product (in pGEMT) by using the forward primer 5'-ATAAGAATGCGGCCGCTATGAATGAGTATCCTAAAA-3' (= FVR662F; Table 1) which contains a NotI restriction site (underlined) and a compatible reverse primer (= FVR663R; Table 1) 5'-CGGATACAGCATAGCGTAGAAAAGGCAGTGTGGTC-3'. The amplified fragment was digested with NotI and AlwNI. The second fragment was obtained by digestion of the initial two-hybrid clone of ANC_2H01 with XhoI and NcoI and ligation of this fragment into the XhoI-NcoI digested vector pJRD184 (Heusterspreute et al., 1985; John Davis, personal communication). This clone was finally used for the isolation of the AlwNI-KpnI fragment of the ANC_2H01 cDNA. Both fragments were ligated into the NotI-KpnI fragment of the vector pEFHOBES. The in-frame cloning was confirmed by DNA sequence analysis with the ANC_2H01-specific primer 5'-TCTGTTTCTGGCCTTGATTC-3' (=FVR310R; Table 1).

Another three points ligation was set up to fuse the ANC_2H01 protein carboxyterminally to the E-tag. The vector used was the pDNATRADE (gift from Dr. W. Declercq and B. Depuydt, Department of Molecular Biology, VIB-University of Gent, Belgium), which was digested with the NotI and HindIII restriction enzymes. The HindIII site was completely filled in. The second fragment was amplified from the initial two-hybrid clone ANC_2H01 using the forward primer 5'-ATCGTCAGCGACATAGGTCAATGGAATTTTCTCTGAT-3' (= FVR660F; Table 1) and the compatible reverse primer 5'-ATAAGAATGCGGCCGCTGTTGTCTCATGGACTGGAAG-3' (= FVR661R; Table 1), containing a NotI restriction site (underlined). The obtained PCR fragment was digested with AlwNI and NotI restriction enzymes. The third fragment was obtained from the digestion of pGEMTRACE1C with Bsu36I and AlwNI. The constructed plasmid was called pDNA-ANC_2H01E.

### Construction of plasmids encoding αN/αE-catenin chimeras fused to the GAL4-DBD.

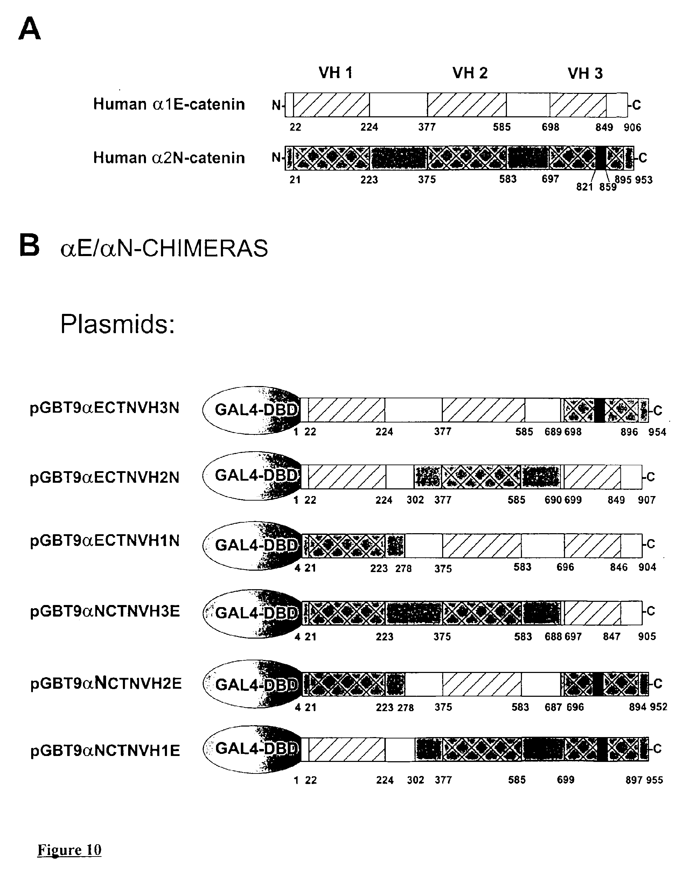

We constructed six different plasmids in which different parts of either αN-catenin cDNA or αE-catenin cDNA were exchanged with the homologous part of, respectively, the αE-catenin cDNA or the αN-catenin cDNA. This was done by a combination of PCR products and restriction fragments. A first fragment was amplified from the pGBT9ANCTN plasmid by using the forward primer 5'-CGGAATTCCCGGGGGCAACTTC-3' (= FVR1241; Table 6) which contains an EcoRI restriction site (underlined), and a compatible reverse primer 5'- TCATTAAGAGCATATGCCAGCT -3' (= FVR1243; Table 6) which includes a new NdeI restriction site (underlined). The PCR product was digested with the appropriate restriction enzymes and subsequently ligated into the EcoRI-NdeI digested pGBT9α ECTN plasmid. This construct was named pGBT9αNCTNVH1E referring to the vinculin-homologous domain 1 (VH1) of α N-catenin that is replaced by that of αE-catenin (Fig. 10) .

To construct pGBT9αECTNVH1N, a fragment was amplified from the pGBT9αECTN plasmid using the forward primer 5'-AATTCCCGGGCGCCCAGCTAGC-3' (= FVR1244; Table 6) comprising an XmaI restriction site (underlined) and a compatible reverse primer 5'-TCCTCCAGGGACGGCCGAAAGC-3' (= FVR1245; Table 6) which includes an EagI restriction site (underlined). Subsequently, the amplified fragment was digested with the appropriate restriction enzymes and ligated into the fragment obtained by XmaI-EagI digestion of pGBT9ANCTN. The constructed plasmid is called pGBT9α ECTNVH1N and contains the cDNA of αE-catenin in which the VH1 domain of αE-catenin is replaced by that of αN-catenin (Fig.10).

The vinculin-homologous domain 2 of αN-catenin (VH2N) was amplified from the pGBT9ANCTN plasmid using the forward primer 5'-AGGTTCCGGCCGTCCCTGCA-3' (= FVR1246; Table 6) and a compatible reverse primer 5'-GGAATATCGGTACCTGCTCAGC-3' (= FVR1247; Table 6), including, respectively, an EagI and a KpnI restriction site (underlined). A second fragment containing the vinculin-homologous domain 3 of αE-catenin (VH3E) was generated by PCR from the pGBT9αECTN plasmid, using the forward primer 5'- AACAGGTACCCAGCTTCCAGG-3' (= FVR1252; Table 6) which includes a KpnI restriction site (underlined) and the reverse primer 5'-CTTGGCTGCAGGTCGACTCT-3' (= FVR1253; Table 6), containing a SalI restriction site (underlined). These two PCR fragments were digested with the appropriate restriction enzymes and subsequently ligated into the EagI-SalI digested pGBT9αNCTNVH1E. The constructed plasmid contains the cDNA of αE-catenin in which the VH2 is replaced by that of αN-catenin and is therefore named pGBT9αECTNVH2N (Fig. 10). These same two digested PCR fragments were ligated into the EagI-SalI digested pGBT9ANCTN plasmid to obtain the pGBT9αNCTNVH3E plasmid, in which the VH3 domain of αN-catenin is replaced by that of αE-catenin (Fig. 10).

A PCR fragment was generated from the pGBT9αECTN plasmid using a forward primer 5'-ACTGGCATATGCACTCAATAAC-3' (= FVR1250, Table 6), containing an NdeI restriction site (underlined), and a compatible reverse primer 5'-CCTGGAAGCTGGGTACCTGTTC-3' (= FVR1251; Table 6), including a KpnI restriction site (underlined). A second fragment was generated by PCR from pGBT9ANCTN using the forward primer 5'-GCTGAGCAGGTACCGATATTCC-3' (= FVR1248F; Table 6) and a reverse primer 5'-TTGGCTGCAGGTCGACGGTATC-3' (= FVR1249R; Table 6), including, respectively, a KpnI and a SalI restriction site (underlined). These two PCR fragments were digested with the appropriate restriction enzymes and ligated into the NdeI-SalI digested pGBT9α ECTNVH1N plasmid to obtain the pGBT9αNCTNVH2N (Fig. 10). To construct the pGBT9αECTNVH3N plasmid, these same two PCR fragments were ligated into the NdeI-SalI digested pGBT9αECTN (Fig. 10).

The in-frame cloning of all the different fragments was confirmed by DNA sequence analysis of the constructs, using primers FVR51, FVR54, FVR157, FVR160, FVR217, FVR738, FVR1157 and FVR1311 (Table 6).

### Construction of plasmids encoding about full-length ANC_2H01 fused to either the GAL4-DBD or GAL4-AD.

To construct the two-hybrid plasmid, which encodes about the full-length ANC_2H01 protein fused to the GAL4-DBD, a three points ligation was set up. The first fragment was amplified from the cloned 5' RACE product (in pGEMT) by using the forward primer 5'-GGAATTCCTGAATGAGTATCCTAAAAAA-3' (= FVR1237F; Table 1) which contains a new EcoRI restriction site (underlined), and a compatible reverse primer 5'-ATGCATGCTGTAGAAAAGGCAGTGTGGT-3'(= FVR1238R; Table 1). The amplified fragment was digested with EcoRI and AlwNI. A second fragment was generated by PCR from the initial two-hybrid clone ANC_2H01 using the forward primer 5'-CGTCGCGGCCCTGCAGATGGATTCAATGGA-3' (= FVR1240F; Table 1) and a compatible reverse primer 5'-TCCCCCCGGGGGGATGAATTTATTATTTTA-3' (= FVR1242R, Table 1), extended with an XmaI restriction site (underlined). The latter PCR fragment was digested with AlwNI and XmaI. Both digested PCR fragments were ligated into the EcoRI-XmaI fragment of the vectors pGBT9 and pGAD424. These constructed plasmids were called pGBT9ANC_2H01FL and pGAD424ANC_2H01FL, respectively. The in-frame cloning of the fragments was confirmed by DNA sequence analysis for both plasmids.

### Construction of plasmids encoding either the aminoterminal or the carboxyterminal part of ANC_2H01 fused to the GAL4-DBD.

A fragment was amplified from the expression vector pEFBOSANC_2H01E using the forward primer 5'-TCCCCCGGGTATGAATGAGTATCCTAAAAAA-3' (= FVR1411F; Table 1), extended with an XmaI restriction site (underlined), and the compatible reverse primer 5'-AAAAGTCGACGGCCACTGCTATTAGCTCTC-3' extended with a SalI restriction site (underlined) (= FVR1412R; Table 1). The fragment was digested with the XmaI and SalI restriction enzymes and ligated into the XmaI-SalI digested pGBT9 vector. The constructed two-hybrid plasmid encodes the aminoterminal, non-zinc finger part of ANC_2H01 fused to the GAL4-DBD and is called pGBT9ANC_2H01-AT.

In order to fuse the carboxyterminal, zinc finger part of ANC_2H01 to the GAL4-DBD, another fragment was amplified from the pDNA-ANC_2H01E plasmid, using the forward primer 5'-GGAATTCTTCTATAAATGTGAACTTTGT-3' (= FVR1413F; Tablel), which includes an EcoRI restriction site, and the compatible reverse primer 5'-AAAAGTCGACAAGTTAAAGAGAATAATCAA-3', extended with a SalI restriction site (= FVR1414R). This PCR fragment was digested with the EcoRI and SalI restriction enzymes and subsequently ligated into the EcoRI-SalI fragment of pGBT9. This plasmid was named pGBT9ANC_2H01-ZF.

### Yeast strains and media

The Saccharomyces cerevisiae strain HF7c (Mata, ura3-52, his3-Δ200, ade2-101, lys2-801, trp1-901, leu2-3, 112, gal4-542, gal80-538, lys2::GAL1-HIS3, URA3::GAL4 17-mers)-CYC1-LacZ) (Matchmaker™, Clontech, Palo Alto, CA) was used for most assays. The HF7c yeast strain carries two reporter genes, HIS3 and LacZ, both integrated into the yeast genome and under the control of GAL4 responsive elements, respectively, the GAL1 UAS and the UAS_{G-17mer}. It has also two auxotrophic markers, trp1 and leu2, which are used for plasmid selection upon transformation. Yeast cultures were grown at 30°C in either complete YPD medium (1% yeast extract, 2% peptone and 2% glucose) or in SD minimal medium (0.5% yeast nitrogen base without amino acids, 2% glucose, and 1% of the appropriate amino acid drop out solution).

The *Saccharomyces cerevisiae* strain Y190 (Mata, ura3-52, his3-200, ade2-101, lys2-801, trp1-901, leu2-3, 112, gal4Δ, gal80Δ, cyh^{r}2, LYS2::GAL1_{UAS}-HIS3_{TATA}-HIS3, URA::GAL1_{UAS}-GAL1_{TATA}-lacZ) (Matchmaker™, Clontech, Palo Alto, CA) was used to perform control experiments. The Y190 strain exhibits a significant level of constitutive leaky expression of the *HIS3* reporter gene. This background can be repressed by including 40 mM 3-aminotriazole (3-AT) in the medium. This strain also contains an integrated LacZ reporter gene under the control of the GAL4 responsive elements in the GAL1 UAS and GAL1 minimal promoter. This results a high level of LacZ expression when induced by a positive two-hybrid interaction. *In vivo* assay on agar plates can be performed with this strain, unlike strain HF7c for which colony transfer to filters is needed (see below).

### Yeast transformation and β-galactosidase filter assay

Plasmids encoding the GAL4 hybrid proteins were introduced into the HF7c yeast reporter strain by the lithium acetate (LiAc) transformation procedure (Gietz et al., 1992, MATCHMAKER Yeast protocol Handbook). Transformants were selected for the presence of the plasmids by growing on appropriate media at 30°C. They were allowed to grow until the colonies were large enough to perform a β-galactosidase filter assay, usually 3-4 days. The transformed cells were then transferred onto a 82-mm nitrocellulose membrane (Sartorius, Goettingen, Germany), permeabilized by freezing the membranes in liquid nitrogen for one minute and followed by thawing at room temperature. The membranes are soaked with 1.5 ml of Z-buffer containing 5-bromo-4-chloro-3-indolyl-β-D-galactosidase (X-gal) and incubated at 30°C until the appearance of blue colonies. This takes usually 30 min to 12 hours. The membranes are then dried, analyzed and stored.

Using the Y190 strain, the β-galactosidase assay was done *in vivo,* on agar plates containing SD minimal medium lacking Trp, Leu and His (1,5 % agar, 0.5% yeast nitrogen base without amino acids, 2% glucose, and 1% of the appropriate amino acid drop out solution), completed with 0.07 M potassium phosphate pH=7, 40 mM 3-AT and X-gal (80 mg/ml).

### Two-hybrid cDNA library screening and DNA sequence analysis

The plasmid pAS2ANCTN was used to screen a human kidney cDNA library, cloned in the GAL4 activation domain vector pGAD10 (Clontech, Palo Alto, CA). The plasmids were introduced into the HF7c yeast strain by using sequential transformation by the lithium acetate (LiAc) method described by Gietz et al. (1992) The interaction screen was carried out in the yeast strain HF7c on media lacking leucine, tryptophan, histidine, and containing 5 mM 3-aminotriazole (3-AT). The β-galactosidase activity in yeast was measured using a β-galactosidase filter assay.

Yeasts harboring interacting proteins were used for plasmid isolation. The obtained plasmid mixture was transformed into Escherichia coli HB101 electrocompetent cells. HB101 has a defect in the leuB gene which can be complemented by LEU2 from yeast. So, this strain can be used for selection of the library plasmid, which carries the yeast LEU2 transformation marker. From these transformants, the library plasmids were isolated and introduced into Escherichia coli DH5α. Plasmids isolated from this strain were further characterized by DNA sequence. This was done by the dideoxy chain termination method (Sanger, 1981) using fluorescent dye terminators in a 373A or a 377 automated DNA sequencer (Applied Biosystems, Foster City, CA). The sequences were further analyzed by BLAST search (Altshul et al., 1990) and the DNAstar software packages (DNASTAR Inc, Madison, USA), and by the Staden gap4 software (Bonfield et al., 1995). To sequence the cDNA insert of the library plasmid, two primers were designed on the pGAD10 vector sequence flanking the cDNA insert: a forward primer 5'-ACCACTACAATGGATGATGT-3' (= FVR174F; Table 2) and a reverse primer 5'-TAAAA-GAAGGCAAAACGATG-3' (= FVR192R; Table 2).

### Northern blot analysis

The total length of the complete mRNA encoding the zinc finger protein was estimated by Northern blot analysis. Total mRNA of various human cell lines was isolated by using RNA-zol B (Wak Chemie-Medical, Bad Homburg, Germany). Total mRNA (15 µg) was glyoxylated, size fractioned on a 1% agarose gel and transferred to a Hybond-N+ membrane (Amersham). The probe was radioactively labeled using random priming (RadPrime DNA labeling system; Gibco BRL Life Technologies, Gent, Belgium). The probe used was a 700-bp BamHI fragment of the isolated two-hybrid clone ANC_2H01, and covered the amino-terminal part and two of the 9 zinc fingers. Hybridizations were performed as described elsewhere (Bussemakers et al., 1991).

### Multiple tissue RNA dot blot analysis

A human RNA Master Blot™ was purchased from Clontech and hybridized according to the manufacturer's instructions. The probe was a 700-bp *Bam*HI restriction fragment of the originally isolated two-hybrid clone ANC_2H01. The labeling was executed by use of the Strip-EZ™ DNA labeling kit (Ambion). Hybridization conditions were as described for the Northern blot analysis. The ExpressHyb™ hybridization solution was purchased from Clontech. The blot was exposed for 7 days to a P-imager screen (Molecular Dynamics).

### Western blot analysis

Cells were lysed by boiling in sample buffer in the presence of 5% β-mercaptoethanol (Laemmli, 1970). The proteins were fractionated by SDS-PAGE and transferred to an Immobilon-P membrane (Millipore, Bedford, MA). The blot was then blocked with 5% nonfat dry milk in PBS^{A} containing 0.01% Tween-20. This was followed by an incubation with the primary antibody for 3 h and after extensive washing, an alkaline phosphatase-conjugated secondary antibody was added for again a 3-hour incubation. Finally, the substrate NBT/BCIP (nitroblue tetrazolium plus 5-bromo-4-chloro-3-indolyl phosphate) for the secondary antibody was then applied to visualize the specific proteins on the membrane. The staining reaction was stopped by rinsing the blot with water.

### Antibodies

The following antibodies were used in Western blot and immunocytochemistry experiments. The monoclonal antibody directed against the E-tag was purchased from Pharmacia and was used in a 1/300 dilution for Western blot analysis and a 1/1000 dilution for immunocytochemistry. The secondary antibody used for Western blot analysis was the anti-mouse alkaline phosphatase-conjugated antibody (dilution 1/500; Sigma Chemical Company, St louis). We used an anti-mouse Cy5-conjugated antibody as a secondary antibody for immunostaining (dilution 1/200, Amersham Pharmacia Biotech, UK).

The polyclonal antibody directed against αN-catenin was purchased from Santa Cruz Biotechnology and used for Western blot analysis (dilution 1/100) as well as for immunocytochemistry (dilution 1/200, Santa Cruz Biotechnology, Inc., California). The secondary antibody applied in Western blot analysis was the anti-goat alkaline phophatase-conjugated antibody (dilution 1/7500, Sigma Chemical Company, St Louis). For immunocytochemistry, we used the anti-goat FITC-conjugated antibody (dilution 1/75, Chemicon International Inc., Temecula, CA).

The polyclonal antibody directed against human and mouse αE-catenin was used for Western blot analysis (dilution 1/4000, Sigma Chemical Company, St Louis). The secondary antibody used was the anti-rabbit alkaline phosphatase-conjugated antibody (dilution 1/5000, Sigma).

To detect E-cadherin in HEK293T cells by Western blot analysis, we applied the mouse HECD1 anti-E-cadherin monoclonal antibody from Zymed (dilution 1/250, Zymed Laboratories, Inc., San Francisco, CA) followed by the secondary anti-mouse alkaline phophatase-conjugated antibody (dilution 1/5000, Sigma).

The anti-Pan-cadherin antibody (dilution 1/500, Sigma), recognizing at least E-cadherin, N-cadherin, P-cadherin, VE-cadherin, R-cadherin and T-cadherin, was also used in Western blot analysis. The secondary antibody used was the anti-rat alkaline phosphatase-conjugated antibody (dilution 1/5000, Sigma).

For the detection of the GAL4 DNA binding domain fusion proteins, a rabbit anti-GAL4 DNA binding domain antiserum was applied (dilution 1/1000; UBI). For the detection of the GAL4 activation domain fusion proteins, a rabbit anti-GAL4 activation domain antiserum was used (dilution 1/1000, UBI). The secondary antibody used in Western blot analysis was anti-rabbit alkaline phosphatase-conjugated antibody (dilution 1/5000, Sigma).

### 5' RACE

In order to complete the cDNA of the clone isolated by the two-hybrid screen, we used 5' RACE technology (GIBCO BRL, Life Technologies, Gent, Belgium). The lacking 5' fragment was isolated using a gene specific primer 5'-GCGGTTCTTCATCAGTTTGG-3' (GSP1 = FVR359R; Table 1) to synthesize the first strand of the cDNA. We performed a PCR with the primer set GSP2 5'-CTCTTGGGTTTGCTGGTTGA-3' (= FVR360R; Table 1) and anchor primer 5'-GAATTCG-TCGACTAGTACGGGIIGGGIIGGGIIG-3' (= FVR239F; Table 3), followed by a nested PCR with the gene specific primer GSP2 (= FVR360R; Table 1) and primer UAP 5'-GAATTCGTCGACTAGTAC-3' (= FVR240F; Table 3). This yielded 3 fragments from human mammary gland mRNA and 2 from human uterus mRNA. No fragments could be amplified from human fetal brain mRNA or human small intestine mRNA. The fragments were further characterized by cloning into pGEM®-T cloning system (Promega, Madison, WI) and by subsequent DNA sequence analysis using the M13 forward (5'-CGCCAGGGTTTTCCCAGTCACGAC-3'; FVR283; Table 5) and M13 reverse primer (5'-TCACACAGGAAACAGCTATGAC-3'; FVR284; Table 5). The specificity of the fragments was determined using the DNAstar software packages (DNASTAR Inc, Madison, USA), and by the Staden gap4 software (Bonfield et al., 1995). The RNA from normal tissue was purchased from Clontech (Clontech, Palo Alto, CA).

### Transfection procedure of HEK293T cells

HEK293T cells (Graham et al., 1977; Wigler et al., 1978) were transiently transfected with the pPNhANCTN and/or the pEFBOSANC_2H01E plasmid by a Ca₃(PO₄)₂ transfection procedure. The cultured HEK293T cells were trypsinized and reseeded at 300.000 cells/ml 24 h before transfection. Two hours before transfection, fresh medium was added to the cells. For a 6-well plate, 2 µg of plasmid DNA per well was used, while for a culture flask of 25 cm² 5 µg of plasmid DNA was applied. The sterile, ethanol precipitated plasmid DNA (purified on a Qiagen DNA purification column, Qiagen Inc., CA, USA) was dissolved in 0.1 x TE (Tris-EDTA) buffer, pH 7.5. The appropriate amount of plasmid DNA was mixed with an equal volume of a mixture comprising 1 volume 125 mM CaCl₂/HEPES, pH 7.05 and 4 volumes 0.1x TE. The DNA mixture was added very slowly to 1x HEPES/2x BS (buffered saline). Upon shaking mildly, the DNA-Ca₃(PO₄)₂ precipitate was formed and could then be added to the medium covering the cells. The incubation of the cells was done at 37°C for 24 h. The DNA-transfection mixture was then removed and replaced by fresh medium consisting DMEM, supplemented with 10% FCS, 0.03% glutamine, 100 U/ml penicillin, 100 mg/l streptomycin and 0.4 mM sodium pyruvate.

### Immunocytochemistry

HEK293T cells were reseeded and grown on glass coverslips and transfected with either the plasmid encoding αN-catenin, or with the plasmid encoding the E-tagged ANC_2H01 protein or with both plasmids. The cells were incubated according to the transfection protocol. When confluent monolayers were formed, cells were fixed with ice-cold methanol for 15 min at -20°C. The cells were washed with PBS^{A}. Subsequently, the cells were incubated for 1 h at 37°C with either a polyclonal antibody against αN-catenin (goat anti-αN-catenin, Santa Cruz Biotechnology), or an antibody against the E-tag (Pharmacia) or both. After extensive washing with PBS^{A}, the appropriate FITC- or Cy5-labeled secondary antibodies were applied for 1 h at 37°C. All antibodies applied were diluted in PBS^{A} containing 0.04% gelatin. Finally, coverslips with cells were mounted with Vectashield (Vector Laboratories, Burlingame, CA). The cells were examined with a Zeiss LSM 410 confocal laser-scanning immunofluorescence microscope (Carl Zeiss, Jena, Germany).

### Raising of polyclonal and monoclonal antibodies against ANC_2H01 protein

We are raising a polyclonal antibody against the ANC_2H01 protein by immunization of rabbits with a ANC_2H01-specific synthetic peptide, that was synthesized and purified by the VIB-Department of Medical Protein Chemistry. This peptide corresponds to the aa residues 73-87 (sequence NH₂-DGKARNRNQNYLVP-COOH) and was chosen on the basis of the Protean program of the DNAstar software packages (DNASTAR Inc, Madison, USA). The peptide was extended with an additional cysteine residue at the carboxyterminus. Via this cysteine residue, the peptide was conjugated to keyhole limpet hemocyanin (KHL, Sigma). Three rabbits were each immunized with 200 µg peptide, mixed with complete Freund's adjuvans. The results of these immunizations are not available yet.

Monoclonal antibodies will be generated using GST-fusion proteins. Several plasmids, containing different fragments of ANC_2H01 fused to the GST-cDNA are constructed. In a first approach, the aminoterminal non-zinc finger part was fused to GST and will be used to generate monoclonal antibodies.

### PAC Screening

The isolation of a human genomic DNA clone comprising the ANC_2H01 gene was done by PCR screening of a PAC clone library RPCI1 (Ioannou and de Jong, 1996). Pools of human PAC library clones were obtained from the UK HGMP Resource Center (Hinxton, Cambridge, UK) and were screened by PCR, using primers FVR513F (Table 1) and FVR514R (Table 1). A 338-bp PCR fragment was amplified on ANC_2H01 cDNA. Out of these pools of human PAC library clones, one positive clone was identified and an aliquot of this clone was streaked out on a LB-agar plate containing kanamycin and single colonies were re-examined by PCR. A positive colony was then grown and used for DNA isolation.

### Fluorescence In Situ Hybridization analysis (FISH)

FISH analysis using PAC clone 167024 specific for the ANC_2H01 gene was performed according to standard procedures (Kievits et al., 1990) with some minor modifications. The DNA of the PAC clone was biotinylated using a BioNick-kit (Gibco BRL) according to the manufacturer's protocol. Fluorescence image results were captured by a Photometrics Image Point CCD camera (Photometrics-GmbH, Munchen, Germany) mounted on a Zeiss Axiophot microscope (Carl Zeiss, Jena, Germany). Image processing was performed and chromosome G-banding was obtained by reverse DAPI-banding using the MacProbe v3.4.1 software (Perceptive Scientific International LTD., League City, TX, USA).

### CASTing

CASTing (Cyclic Amplification and Selection of Target sequences (Wright et al., 1991) will be used to determine any DNA sequences to which the ANC2H01 protein binds specifically. The first step is the construction of a plasmid to obtain the protein of interest fused to GST. Using this GST-part, the protein can be immobilized on a gluthatione column. We have cloned the zinc finger domain of ANC_2H01 into the pGEX4T vector (Pharmacia).

### RESULTS

### Two-hybrid cDNA library screening

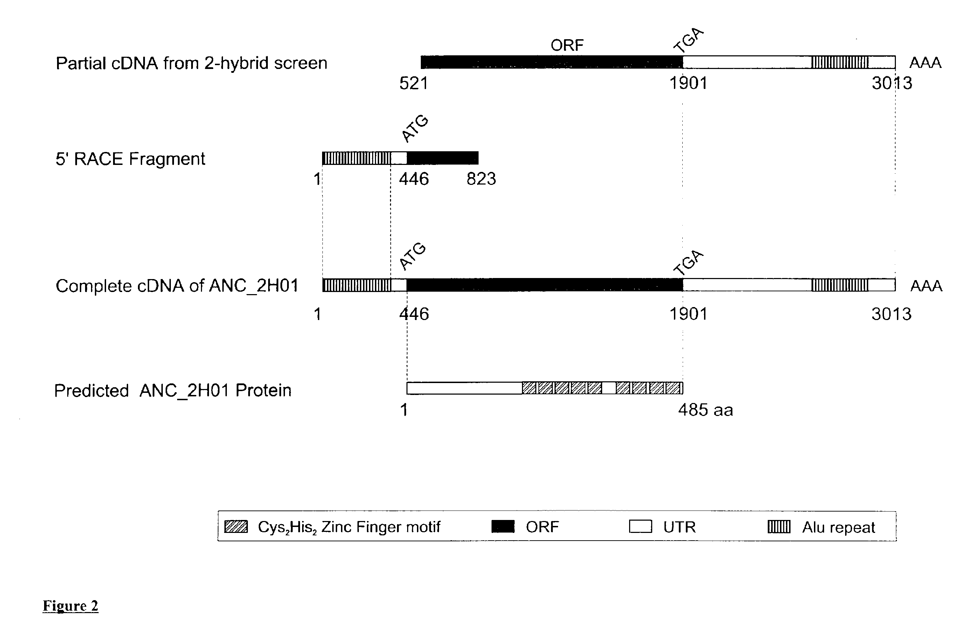

For the initial two-hybrid screen, almost the full-length αN-catenin cDNA was fused to the GAL4 DNA binding domain in the pAS2 vector (Clontech) and assayed for interaction with proteins encoded by a GAL4 activation domain cDNA library from human kidney (Clontech). About 3x10⁵ clones were screened and examined for interaction with αN-catenin on the basis of induction of two genes: the selection gene HIS3 and the reporter gene LacZ. One clone, that exhibited the desired HIS3-positive, β-galactosidase-positive phenotype, was isolated out of this screen and was further examined. The clone contained an insert of about 2,500-bp (Fig. 2). The cDNA insert of the clone was completely sequenced using universal and specific walking primers (see Table 1). The cDNA insert revealed an open reading frame (ORF) of 459 amino acids. The insert contained a stop codon, a poly-A signal, a poly-A tail, but no start codon could be detected. Analysis of the encoded protein revealed the presence of 9 Cys₂His₂ zinc fingers at the carboxy-terminal half of the protein (Figs. 1 to 3). Zinc fingers of the Cys₂His₂-type, originally discovered in TFIIIA (Hanas et al., 1983; Miller et al., 1985), often function in nucleic acid binding and, more particularly, in sequence specific recognition of DNA, which is pivotal for the function of transcription factors. The 3' untranslated region (UTR) contained an Alu repeat region of 286 nucleotides (Figs. 1 and 2). The two-hybrid clone isolated in this screen was called ANC_2H01 (for Alpha-N-catenin 2-hybrid clone 01) .

### Retransformation of ANC_2H01 in the two-hybrid system

The primary purpose of this test is to check the specificity of the interaction between ANC_2H01 and αN-catenin. The isolated two-hybrid clone was retransformed into the HF7c strain. This ANC_2H01 clone was combined with either one of the following: the original bait plasmid pAS2ANCTN, the empty vector pAS2, the pGBT9ANCTN plasmid, the empty vector pGBT9 and pLAM5', a plasmid encoding an irrelevant protein (lamin C) fused to the GAL4 DNA binding domain. The ANC_2H01 exhibited the desired HIS3-positive β-galactosidase-positive phenotype upon combination with either pAS2ANCTN or pGBT9ANCTN. No interaction was seen when combination with either one of the empty vectors pGBT9 or pAS2, nor when combined with pLAM5'. Therefore, the initial observation was confirmed, which means that the interaction between ANC_2H01 and αN-catenin might be specific.

### Delineation of the domains mediating the interaction between ANC_2H01 and αN-catenin proteins

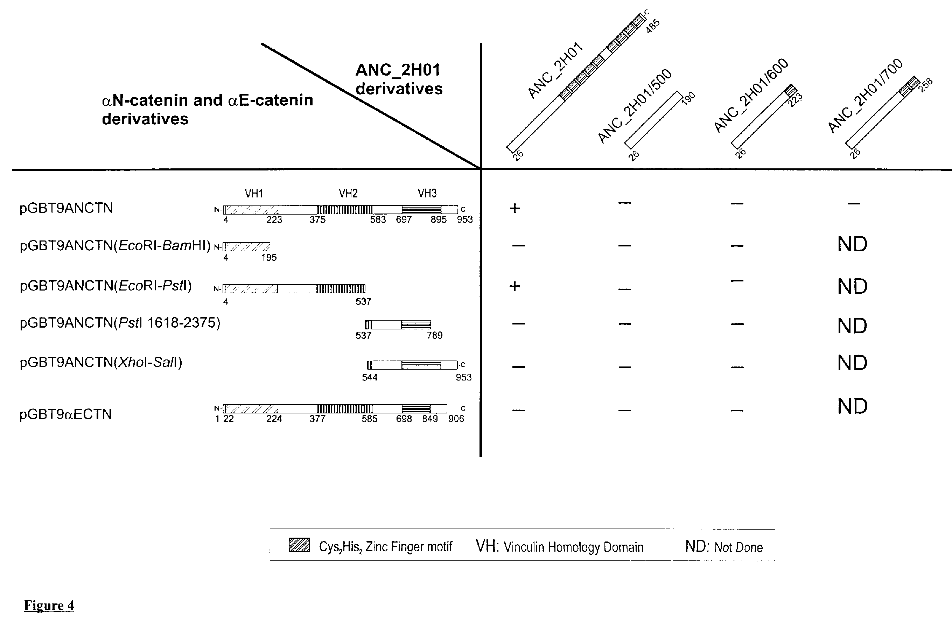

By the use of the two-hybrid system, we tried to analyze which part of the ANC_2H01 protein is responsible for the interaction with αN-catenin. Three different fragments of the initial two-hybrid clone ANC_2H01 were subcloned in the pGAD10 vector and were designated, respectively, ANC_2H01/BamHI, ANC_2H01/600 and ANC_2H01/500 (Fig. 4). These three cDNA fragments each include the amino-terminal part of ANC_2H01. No interactions, however, could be observed between these different parts of ANC_2H01 and the αN-catenin protein (Fig. 4).

By cloning different parts of αN-catenin into the pGBT9 vector, we delineated the domain responsible for association with ANC_2H01 to amino acid residues 4-537 of αN-catenin. The transformants harboring the plasmid pGBT9ANCTN(EcoRI-PstI) together with the ANC_2H01 plasmid exhibited the HIS3-positive, β-galactosidase-positive phenotype in the two-hybrid system (Fig. 4). All other combinations of αN-catenin fragments and ANC_2H01 did not result in the expression of the two reporter genes. Besides the interaction with the initial ANC_2H01 clone, we also looked in the two-hybrid system for the interaction of the various αN-catenin fragments with ANC_2H01/BamHI, ANC_2H01/600 and ANC_2H01/500. However, we could not observe any interaction between the different fragments of αN-catenin and any of the truncated ANC_2H01 fusion proteins (Fig. 4).

As αE-catenin is an isoform of αN-catenin, the cDNA for αE-catenin was cloned in frame with the GAL4 DNA binding domain into the pGBT9 two-hybrid vector. This clone was then assayed for interaction with ANC_2H01 and truncated derivatives in the two-hybrid system. However, no interaction was observed when both plasmids were cotransformed into the HF7c strain (Fig. 4).

### Northern Blot Analysis

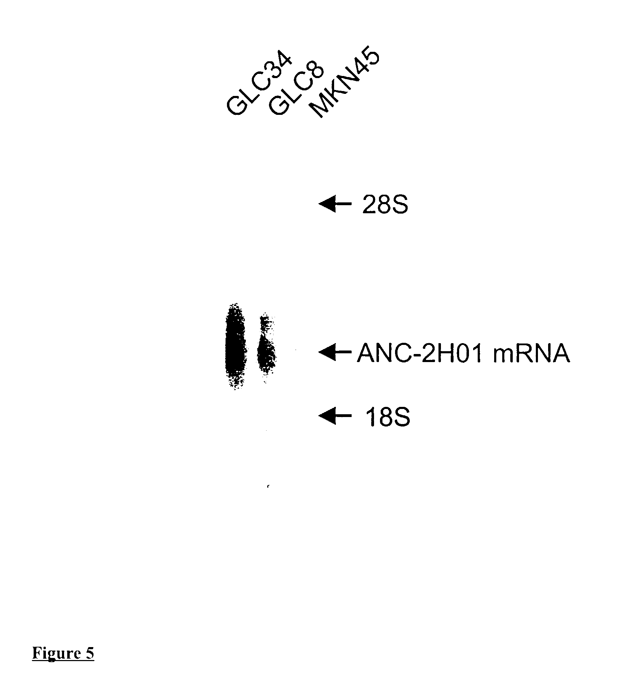

In order to estimate the length of the complete mRNA of ANC_2H01, we performed a Northern blot analysis. Total RNA of various human cell lines was hybridized with a ³²P-labeled 700-bp BamHI fragment of the ANC_2H01 cDNA. A very weak signal could be detected in some cell lines, but most of the lanes lacked specific signal. However, a stronger signal was seen in the lanes with GLC34 and GLC8 RNA (Fig. 5). The positions of 28S and 18S ribosomal RNA on the blot were visualized using methylene blue staining. Using these positions as markers, we could estimate the size of the ANC_2H01 mRNA to be about 3,000 bp. The cDNA insert of the original ANC_2H01 two-hybrid clone was about 2,500 bp, and contained a stop codon, a poly-A signal and a poly-A tail (Fig. 2). However, a start codon could not be detected. This implies that we were lacking a 5' fragment of about 500 bp.

### Multiple tissue RNA dot blot analysis

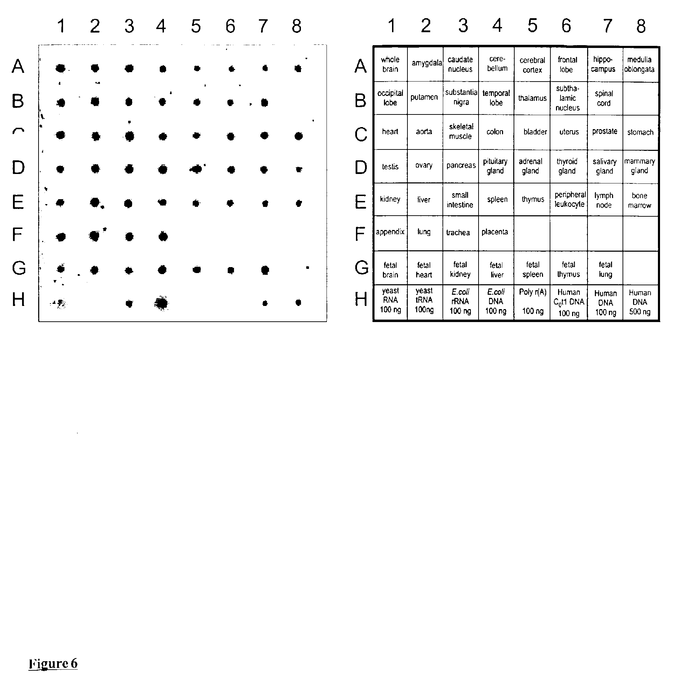

To survey tissue-specific expression of ANC_2H01, a multiple tissue RNA dot blot analysis was performed with a commercially available human RNA dot blot (Clontech). The ANC_2H01 mRNA showed a ubiquitus expression (Fig. 6). However, the strongest expression was observed for pituitary gland and adrenal gland mRNA. Lung, placenta, fetal liver and fetal lung also showed increased expression of the mRNA.

### 5' RACE

To isolate the lacking 500-bp 5' fragment of the ANC_2H01 cDNA, we used the 5' RACE technology (Gibco BRL). For this purpose, we used human mRNAs derived from mammary gland, fetal brain, uterus and small intestine (Clontech). Using a gene specific primer , an ANC_2H01 specific cDNA strand was synthesized. Using two nested primers sets (set #1 and set #2; see Table 1), we isolated 5 fragments from mammary gland and uterus mRNA. No fragments were amplified using the fetal brain or the small intestine mRNA. The 5' RACE fragments were cloned into the pGEM®-T cloning system and characterized by DNA sequence analysis. One of the inserts derived from mammary gland mRNA turned out to be specific for ANC_2H01. The four other 5'RACE products were nonspecific. From further sequence analysis using walking primers (see Table 1) the size of the 5' RACE fragment was determined to be 823 bp (Fig. 2). This 823-bp fragment contained 520 bp as new 5' sequence and 303 bp as contained within the initial ANC_2H01 two-hybrid clone (Fig. 2). The 5' RACE fragment contained a start codon and 26 more codons that were not covered by the original ANC_2H01 two-hybrid clone (Fig. 1). In addition to the Alu repeat region detected in the 3'UTR of the cDNA, the 5'UTR also contains an Alu repeat region (Fig.1 and Fig. 2).

### The ANC_2H01 gene product is a zinc finger protein

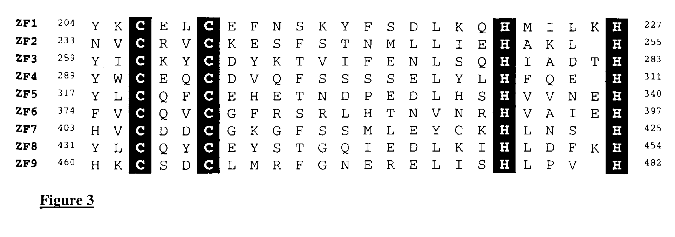

Scrutiny of the entire sequence of the ANC_2H01 cDNA revealed several interesting features. The cDNA has a total length of 3,013 bp. The 5'UTR was as long as 445 nt. The context of the start codon suits an adequate Kozak initiator sequence (Kozak, 1996). The 3' region contains a poly-A signal and a poly-A tail, indicating that also the 3'UTR is completely isolated and has a size of 1,112 nt. The 3'UTR as well as the 5'UTR contain one Alu repeat region (see Fig. 1). The cDNA encodes a protein of 486 amino acid residues (aa). The deduced molecular mass of the protein is 53.46 kDa. The protein contains nine zinc fingers in its carboxy-terminal half. All of these zinc fingers are of the Cys₂His₂ type (Fig. 3). They are clustered into two domains and followed by 3 aa only before the translational stop codon. The first five Cys₂His₂ zinc fingers are separated from the last four zinc fingers by 33 aa. Within each cluster, each zinc finger motif is only 3 to 5 aa apart from the following zinc finger. The presence of the 9 Cys₂His₂ type zinc fingers indicates that the protein binds double stranded DNA. In addition, a nuclear localisation signal (PKKRKRK; for review Görlich and Mattaj, 1996) is present in the protein at the amino-terminal side (residues 5-11; Fig. 1).

Further characterization of the isolated clone included a BLASTN search analysis (Altschul et al., 1990). The complete cDNA of ANC_2H01 did not correspond with any cloned full-size cDNA in the public-domain databases. However, several EST (Expressed Sequence Tag) clones with high degree of sequence similarity (low P-score) are present in the databases. These ESTs are from human origin (Table 7), as well as mouse origin (Table 8).

### The ANC_2H01 protein localizes to the nucleus and translocates αN-catenin into the nucleus

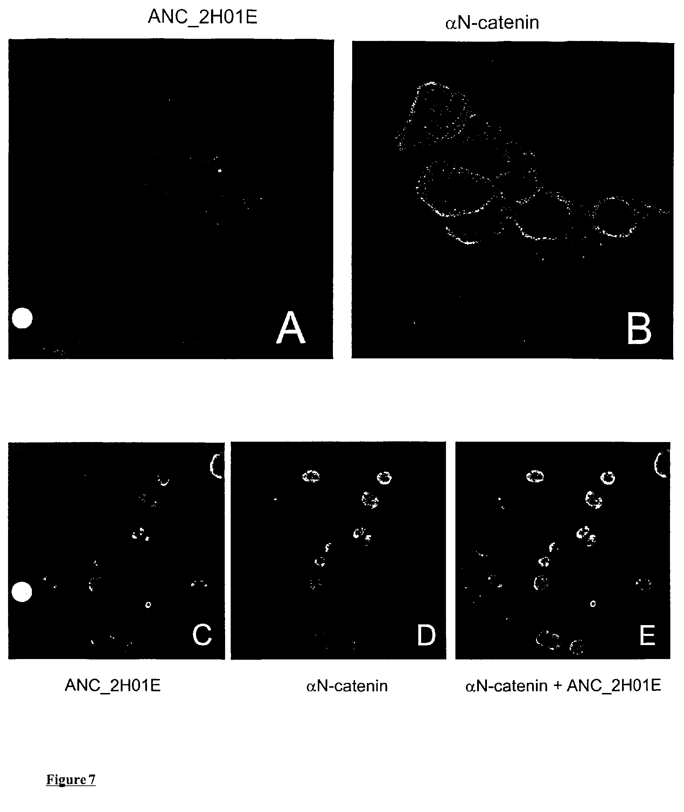

HEK293T cells were transiently transfected with the pPNhANCTN plasmid and/or the pEFBOSANC_2H01E plasmid by a Ca₃(PO₄)₂ transfection procedure. The subcellular localization of the αN-catenin and the ANC_2H01 protein were analyzed by immunocytochemistry. The epitope tagged zinc finger protein could be specifically stained with a monoclonal antibody against the E-tag. To detect αN-catenin a commercial polyclonal antibody was used. The zinc finger protein ANC_2H01 localized to the nucleus upon transient expression in HEK293T cells (Fig. 7A). This nuclear staining was seen as well in cells transfected with the pEFBOSANC_2H01E plasmid alone as in cells that were transfected with both expression plasmids. In contrast, the αN-catenin protein was localized into the cytoplasm of cells transfected with plasmid pPNhANCTN only (Fig. 7B). When combined with the expression plasmid encoding the E-tagged ANC_2H01 protein, αN-catenin localized to the nucleus (Fig. 7C-E). So, a translocation of αN-catenin into the nucleus occurs, when the interacting zinc finger protein ANC_2H01 was co-expressed.

### Western Blot Analysis

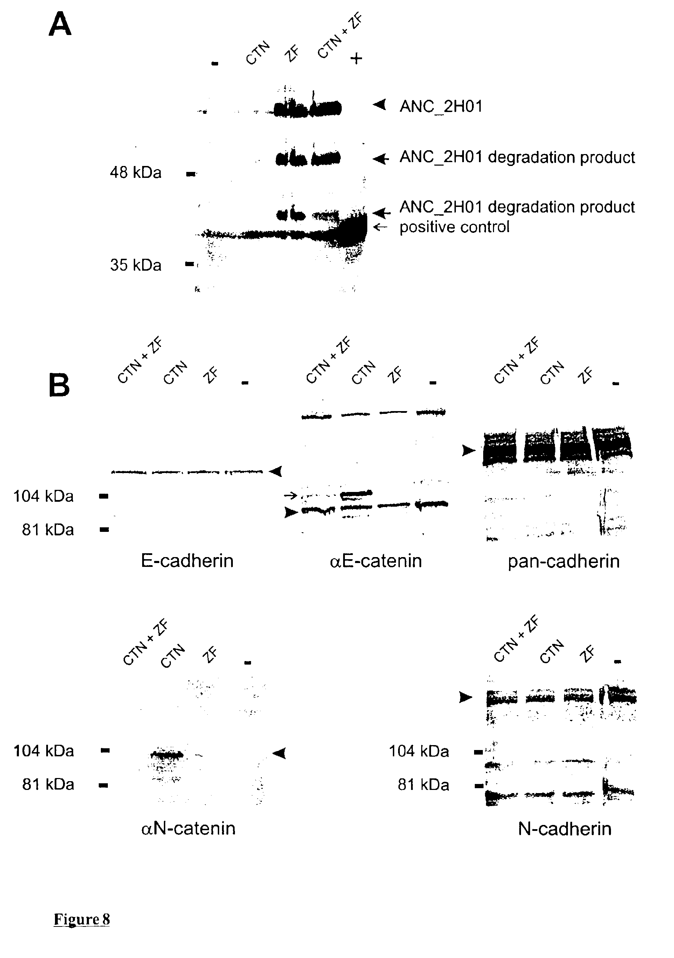

Lysates of HEK293T cells transiently transfected with pPNhANCTN and/or pEFBOSANC_2H01 were prepared 24 h after transfection and examined for the expression of the recombinant proteins. The ANC_2H01 protein was detected by the use of the E-tag. The tagged protein has a predicted molecular mass of approximately 55 kDa and such a band was indeed detectable (Fig. 8A). Two weaker bands with lower molecular weight were also observed on the blot. This may be due to proteolysis of the protein at the carboxy-terminus. The E-tag, which is fused at the amino-terminal part of the protein, is still available in protein fragments degraded at the carboxy-terminus.

The presence of αN-catenin was revealed by the use of a polyclonal antibody against αN-catenin. A single band of approximately 100 kDa was recognized by this antibody in single and double tranfected cells (Fig. 8B). The predicted molecular mass of αN-catenin is 100,686 daltons.

The presence of other cadherins and cadherin-associated molecules was also examined in these cells. E-cadherin was present in non-transfected cells as well as in single and double transfected cells (Fig. 8B). Using an anti-pan-cadherin antibody, a broad band could be observed for the different transfected cells, probably covering several cadherins. We also tried to detect αE-catenin, using an antibody against this protein. The transfected as well as the non-transfected cells showed staining for αE-catenin (Fig. 8B). When recombinant αN-catenin was expressed, the anti-αE-catenin antibody recognized also a larger band. This additional band was observed in the αN-catenin single transfectant, but was weaker in the double transfectant. We concluded that the anti-αE-catenin antibody used recognized also the recombinant αN-catenin expressed in these cells.

At a semi-quantitative level we could not detect any drastic differences in the untransfected versus the transfected cells upon staining for E-cadherin, pan-cadherin, or αE-catenin.

### PAC screening

Superpools and subsequent plate-pools from the UK-HGMP PAC library (Roswell Park Cancer Institute, Buffalo, NY) were screened by PCR with ANC_2H01-specific primers FVR513F and FVR514R (Table 1). This resulted in the determination of the plate number of a 384-well microtiter plate containing a positive clone. Further PCR analysis of the pooled rows and columns pinpointed the positive clone 167024 as specific for the ANC_2H01 gene.

### Chromosomal localization of the ANC_2H01 gene by FISH analysis

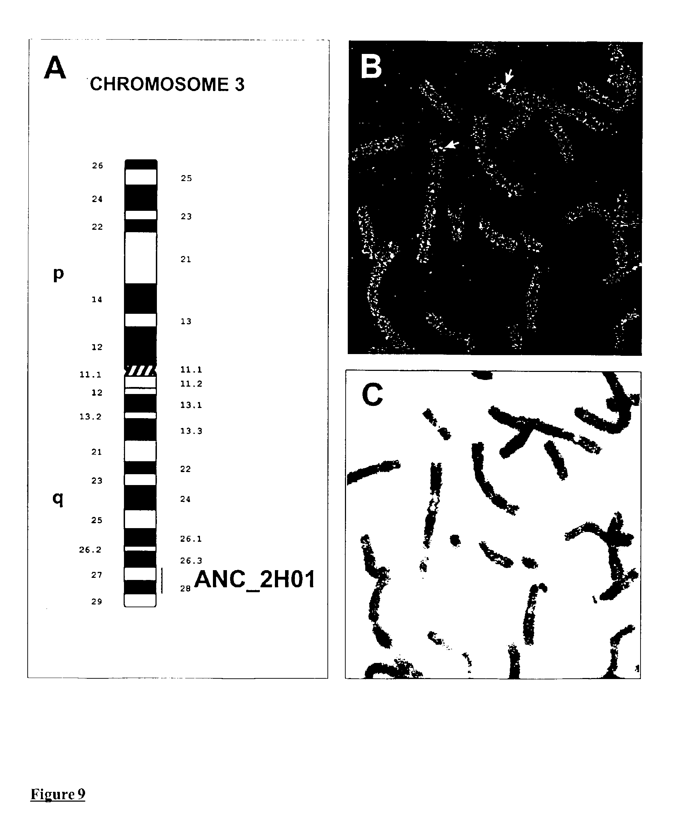

FISH was performed using the PAC clone 167024 specific for the ANC_2H01 gene. We mapped the gene encoding the zinc finger ANC_2H01 on the chromosomal locus 3q27-28. To this end, we analyzed 20 metaphases. Up to 15 of them revealed discrete hybridization signals on both chromosomes 3 at band 3q27-28 (Fig. 9). The other metaphases showed signal on only one of both chromosomes 3.

### Two-hybrid analysis of the interaction of ANC_2H01 with α E/αN-catenin chimeras

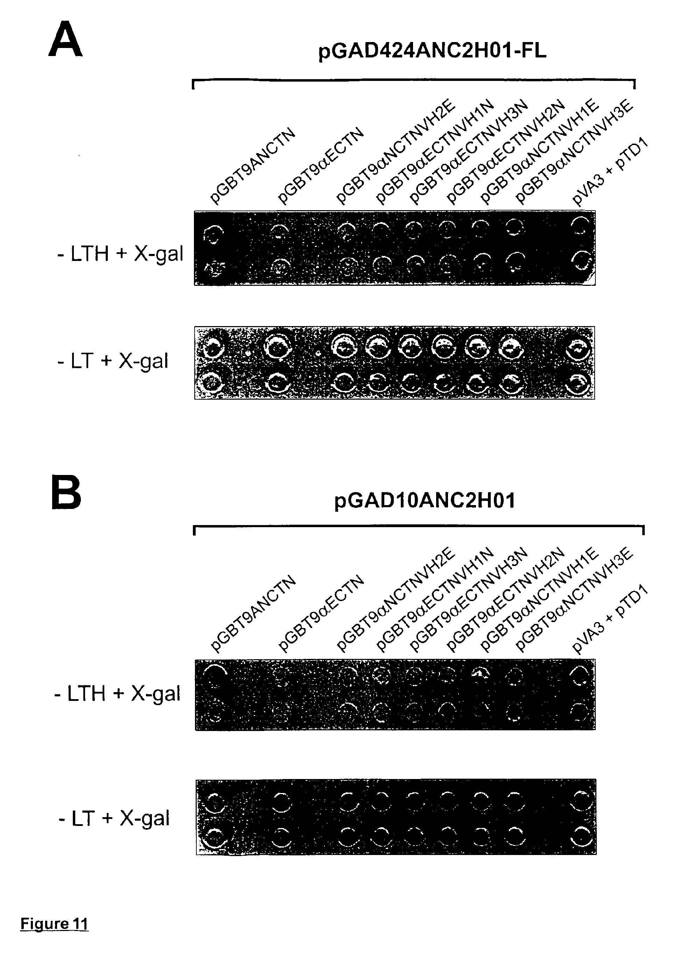

The zinc finger protein ANC_2H01 does not interact with αE-catenin when tested in the yeast two-hybrid system. The human αE-catenin protein shows, however, 80 % homology with the closely related human αN-catenin protein. In spite of this high similarity, their tissue distribution is clearly distinct. Both proteins show also identity with vinculin in three regions with higher sequence conservation, the so-called vinculin homology domains (VH) (Herrenknecht et al., 1991) (Fig. 10). In an approach to further characterize the specific interaction between αN-catenin and ANC_2H01, we made α-catenin chimeric molecules by exchanging these VH domains between αN-catenin and αE-catenin. We constructed six different α N/αE-chimeras fused to the GAL4 DNA binding domain (Fig. 10). The possible interaction of these six αN/αE-chimeras with the ANC_2H01 protein was analyzed using the two-hybrid system. We observed no expression of either the selection gene HIS3 or the reporter gene LacZ when either the initial ANC_2H01 two-hybrid clone or pGAD424ANC_2H01-FL was cotransformed into Y190 yeast cells with pGBT9α ECTN, pGBT9αECTNVH1N, pGBT9αNCTNVH2E or pGBT9αECTNVH3N (Fig. 11). In addition, no interaction could be detected when the Y190 yeast strain was cotransformed with the initial ANC_2H01 two-hybrid clone or pGAD424ANC_2H01-FL on the one hand, and the pGBT9αNCTNVH1E and pGBT9α ECTNVH2N plasmids on the other hand. However, a HIS3-positive, β-galactosidase-positive phenotype was obtained upon combination of ANC_2H01 or pGAD424ANC_2H01-FL with either pGBT9ANCTN or pGBT9αNCTNVH3E (Fig. 11).

### DISCUSSION

For a better understanding of the respective role of the two subtypes of α-catenin protein, we set up a two hybrid screen with human αN-catenin as a bait. The aim was the isolation of proteins that interact specifically with αN-catenin. From a human kidney cDNA library, a single clone was isolated exhibiting the desired HIS3-positive, β-galactosidase-positive phenotype. This two-hybrid clone was completely sequenced and the partial cDNA was completed by 5' RACE. This resulted in a full size cDNA of 3,013 nucleotides (nt) in correspondence with the results of the Northern blot analysis. The 3'UTR as well as the 5'UTR contain an Alu repeat region. In addition, the 3' region contains a poly-A signal and a poly-A tail, indicating that the 3'UTR is completely isolated.

The ATG start codon occurs in an adequate, although not fully optimal context according to Kozak (Kozak, 1996). The open reading frame is 1458 nt in length and encodes a protein of 486 aa. Structurally, the protein can be divided into two domains. The amino-terminal part contains no obvious protein motifs, except for a stretch of basic aa nearby the amino-terminus of the protein (PKKRKRK, aa 5-11). This short sequence resembles the nuclear localization signal of the SV40 large T antigen (PKKKRKV) (Kalderon et al., 1984) and might display the same role in the ANC_2H01, i.e. targeting of the protein to the nucleus. On the other hand, the carboxy-terminal part consists of 9 zinc fingers of the Cys₂His₂ type. These are clustered in two domains, separated from each other by 33 aa. Between consecutive zinc fingers, generally five aa are present. Zinc finger motifs of the Cys₂His₂ type were first discovered in the transcription factor TFIIIA and have DNA binding properties (Hanas et al., 1983; Miller et al., 1985). Another feature of these motifs is their autonomous folding up and their stabilization by chelation of zinc between a pair of cysteine and a pair of histidine residues. It has also been shown that in these polydactyl proteins the DNA interaction is dominated by only few of the many zinc fingers (Sun et al, 1996; Georgopoulos et al., 1997). One zinc finger of the Cys₂His₂ type binds typically 3 bp of a double-stranded DNA sequence, and this is called a subsite. Structural studies of such DNA-protein complex also revealed that consecutive fingers of a polydactyl protein interact with subsites directly adjacent to each other (Pavletich and Pabo, 1991 and 1993). It was also suggested that not all of the zinc fingers from a polydactyl protein contribute to DNA binding and recognition and that the remaining non-DNA-binding zinc fingers may participate in protein-protein interactions. Interactions with another protein as well as homodimerization have been reported in this context (Sun et al., 1996; Morgan et al., 1997).

In this study, we could also show that several amino-terminal fragments of ANC_2H01 do not interact with αN-catenin, at least not in the two-hybrid system. These results may suggest that the amino-terminal domain is not responsible for the interaction. It can also be explained by inappropriate folding of the truncated proteins. In the first case, we should seek an interacting domain in the two zinc finger clusters or in the space of 33 aa in between. Using the two-hybrid system, the associating domain of αN-catenin for ANC_2H01 could be delineated at aa 193-535. In addition, the interaction of αE-catenin with the ANC_2H01 protein could not be observed in the two-hybrid system. Further studies using αE/αN-catenin chimeras showed that for the interaction with the ANC_2H01 protein, the first two vinculin homology (VH) domains of αN-catenin are needed. No interaction was observed when only the VH1 or the VH2 domain of αN-catenin was included in chimeric proteins with αE-catenin. In agreement with the aforementioned data, these results strengthen the conclusion that the interaction with the ANC_2H01 is αN-catenin-specific.

To localize the novel ANC_2H01 zinc finger protein and the αN-catenin/ANC_2H01 complex within cells, we transiently transfected expression constructs encoding these two proteins into human cells (HEK293T) and visualized the proteins by immunofluorescense. The αN-catenin was distributed throughout the cytoplasm, as reported previously (Shibuya et al., 1996). This suggests that the αN-catenin molecules are generally dissociated from cadherins, because cadherins were detected in the lysates of the HEK293T cells. Co-expression of the cDNA encoding the tagged zinc finger protein, resulted in the translocation of αN-catenin to the nucleus. The ANC_2H01 protein was consistently detected in the nucleus. This observation is in line with the presence of a putative nuclear localization signal in the ANC_2H01 protein and with the putative DNA binding nature of the zinc fingers.

Using FISH the ANC_2H01 gene could be mapped to the q27-28 region of human chromosome 3. A study of James and coworkers (James et al., 1996) using YACs covering the human chromosomal regio 3q27, identified ESTs for five genes, including three members of the cystatin gene family and a gene thought to be involved in B-cell non-Hodgkin lymphoma. The latter was confirmed by the identification of the BCL6 (B-cell non-Hodgkin lymphoma) disease gene in this region (Chaganti et al., 1998). The p63 gene, which bears strong homology with the tumor suppressor gene p53 and p73 is also localized in the chromosomal region 3q27-29 (Yang et al., 1998). In addition, another member of this family, p73L, was also assigned to the 3q27-28 region (Senoo et al., 1998).

Taken together, these data show that αN-catenin regulates gene expression by a direct interaction with a novel nuclear zinc finger protein. This transcription factor, that was cloned by us, shows no identity with any cDNA or protein in the public databases, except for a number of unspecified EST clones. The ANC_2H01 protein induces a translocation of αN-catenin to the nucleus by protein-protein association, an observation that has not been reported before. The interaction of the zinc finger protein is considered to be αN-catenin-specific. It is now possible to elucidate the target genes of the transcription factor and the function of the αN-catenin/ANC_2H01 complex in cell-cell adhesion, cellular differentiation and other signalling pathways. Furthermore, it is possible to further delineate the interacting domains of ANC_2H01 and αN-catenin using the two-hybrid system and other approaches; to determine the DNA sequences to which the ANC_2H01 protein specifically binds; to identify other proteins, specifically interacting with the ANC_2H01 protein; to raise specific monoclonal and polyclonal antibodies for the ANC_2H01; and for example to perform drug screens that either enhance or reduce the interaction between the ANC_2H01 and αN-catenin or other proteins.

### BRIEF DESCRIPTION OF THE DRAWINGS

**Figure 1:** Sequence of the full-size human ANC_2H01 cDNA. Dots separate blocks of 10 nucleotides. Sequences of primers used in the 5' RACE experiment are indicated by named arrows. The predicted amino acid sequence of the ORF is indicated in *bold* (one-letter code). The start codon is boxed in and is preceded by an in-frame stop codon (shaded box). The Alu repeat regions in the 5'UTR and the 3'UTR are shaded and boxed in. Also indicated are the nine zinc finger motifs. The amino acid sequence of the peptide used to raise polyclonal antibodies is underlined. The sequence of the putative NLS is also boxed in.

**Figure 2:** Cloning of the full-size cDNA encoding the ANC_2H01 protein.
**ORF** = Open Reading Frame; **UTR** = Untranslated region; **ATG** = start codon; **TGA** = stop codon; **AAA** = poly A tail; **aa** = amino acid residues

**Figure 3:** Alignment of the nine zinc finger motifs (ZF) of the ANC_2H01 protein. Numbers indicate the number of the codon. The amino acids are in the one-letter code.

**Figure 4:** Analysis of interaction of different parts of α N-catenin and full-length αE-catenin with ANC_2H01and different parts thereof. **VH**: Vinculine Homology domain; **ND**: Not Done.

**Figure 5:** Northern blot analysis of ANC_2H01 mRNA in three human tumor cell lines. 28S and 18S are ribosomal size markers.

**Figure 6:** The expression of ANC_2H01 mRNA in various human tissues was studied by use of a human RNA master blot (Clontech). Hybridization was as described in Materials and Methods.

**Figure 7:** Immunocytochemistry of HEK293T cells transfected with either pPNhANCTN (A) or pEFBOSANC_2H01E (B) or with both plasmids (C-E). Tagged ANC_2H01(A) The nuclear staining for the tagged ANC_2H01 protein was visualized using an antibody against the E-tag. (B) The cells were stained with an polyclonal antibody against α N-catenin. The αN-catenin protein is localized in the cytoplasm of the cells. (C) Upon coexpression with αN-catenin, the ANC_2H01 protein remains localized in the nucleus. (D) Upon cotransfection, αN-catenin is translocated into the nucleus. (E) Double immunostaining for the ANC_2H01 and αN-catenin protein was done with the antibodies applied in (A) and (B). Bar: 12 µm.

**Figure 8:** Western blot analysis of transfected and untransfected HEK293T cells. The cells were transfected with either pPNhANCTN (indicated by CTN) or pEFBOSANC_2H01E (indicated by ZF) or with both plasmids. Molecular weight markers are indicated each time at the left. (A) Blots were stained with the monoclonal antibody against the E-tag. A band of approximately 60 kDa is detected (closed arrowhead), which is the expected size for the tagged ANC_2H01 protein. Two weaker bands are also detected and are thought to be degradation products (closed arrows). As a positive control, an unrelated E-tagged protein was loaded in the lane indicated with +. (B) The expression levels of several proteins of the cadherin-catenin complex (indicated by closed arrowheads) were also analyzed in these cells (E-cadherin, N-cadherin, pan-cadherin, αN- and αE-catenin), but no differences could be observed at a semi-quantitative level. The band indicated with an open arrow is presumably αN-catenin, what indicates that the anti-αE-catenin antibody used is cross-reacting with αN-catenin.

**Figure 9:** Chromosomal localization of the human ANC_2H01 gene at band 3q27-28 by fluorescence in situ hybridization. (A) Ideogram of chromosome 3; (B) Fluorescence signal specific for the ANC_2H01 gene; (C) DAPI staining of the same chromosomes.

**Figure 10:** (A) Schematic representation of the vinculin homology domains (VH) in the α1E-catenin and the α2N-catenin protein. α2 isoforms differ from theα1 isoforms by an aternatively used exon nearby the carboxyterminus (black box). (B) Schematic representation of the coding potential of the αE/αN-chimeric cDNAs inserted into the pGBT9 two-hybrid vector. **GAL4-DBD** = GAL4 DNA-binding domain

**Figure 11:** The Saccharomyces cerevisiae strain Y190 was cotransformed with: (A) plasmid pGAD424ANC2H01-FL encoding full-length ANC_2H01 fused to the GAL4-AD, in combination with a plasmid encoding αN-catenin, αE-catenin or αE/αN-catenin chimeras fused to the GAL4-DBD (Fig. 10), and (B) the initial two-hybrid clone pGAD10ANC2H01 encoding part of ANC_2H01 fused to the GAL4-AD, in combination with plasmids encoding αN-catenin, αE-catenin or αE/αN-catenin chimeras fused to the GAL4-DBD (Fig. 10). The plates contained the medium composition indicated at the left completed with 40 mM 3-AT and 80 mg/ml X-gal.
**GAL4-AD** = GAL4 transcription activation domain; **GAL4-DBD** = GAL4 DNA-binding domain; **-LT** = SD medium lacking Leu and Trp; **-LTH** = SD medium lacking Leu, Trp and His. **pVA3** and **pTD1** are plasmids used as a positive control in the two-hybrid system (Matchmaker™, Clontech).

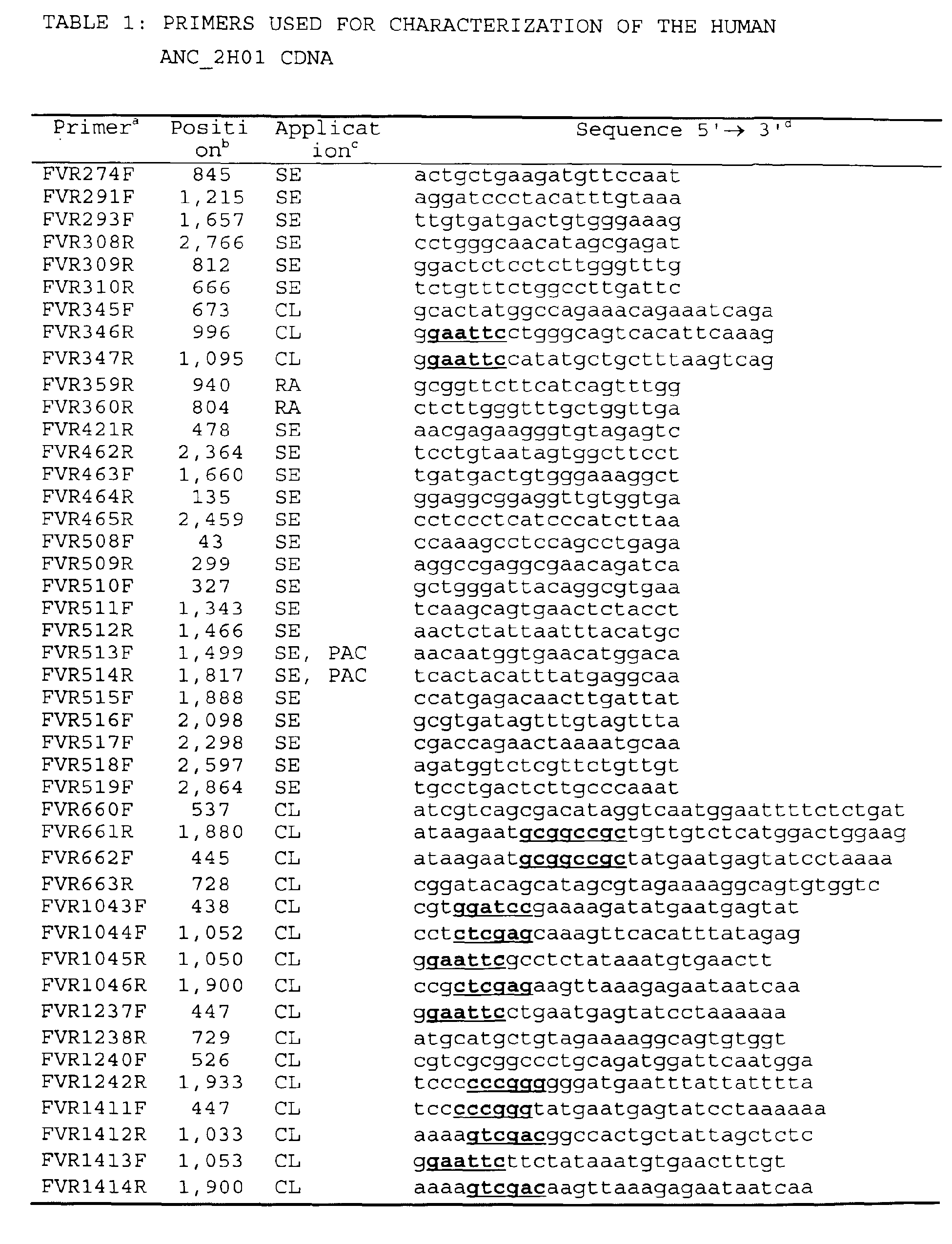

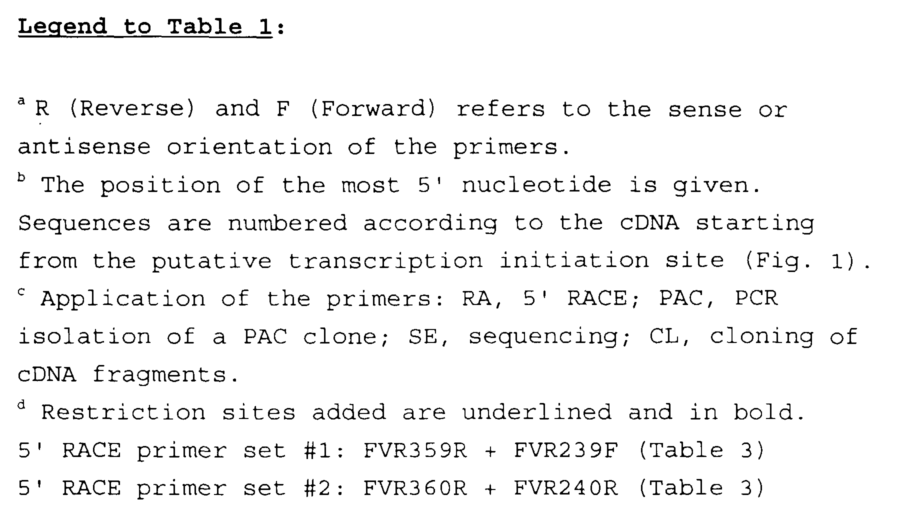

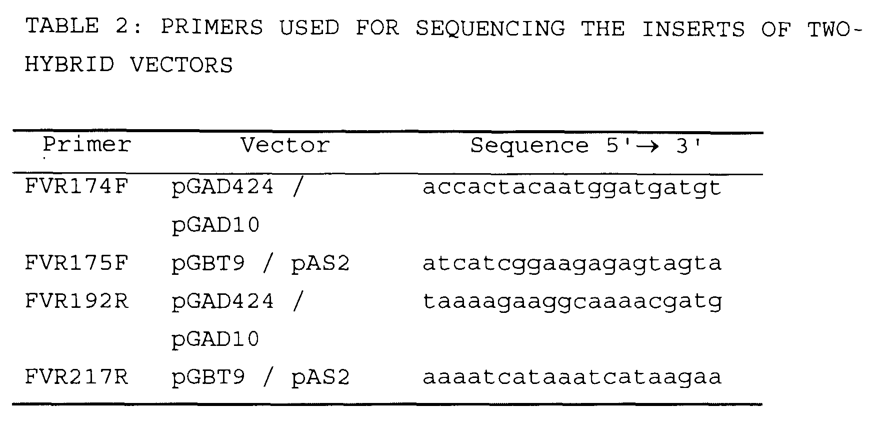

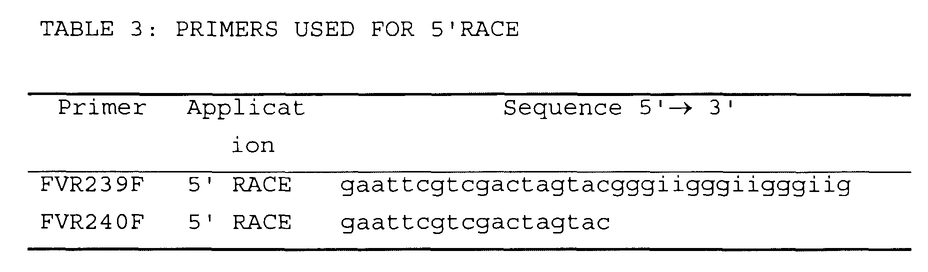

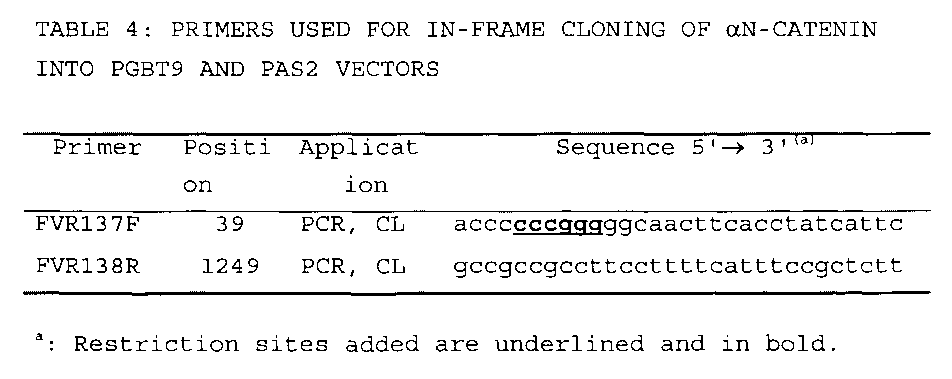

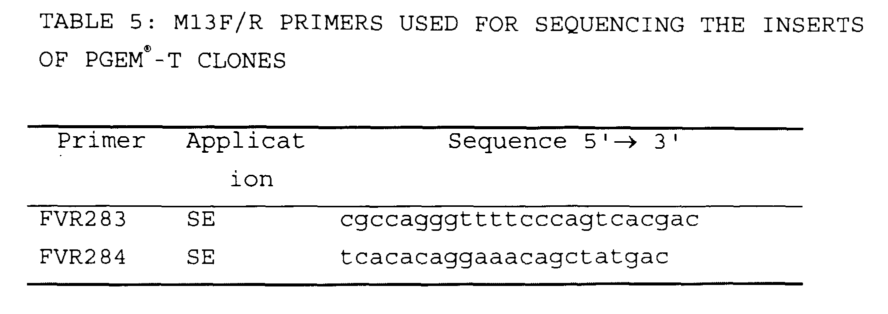

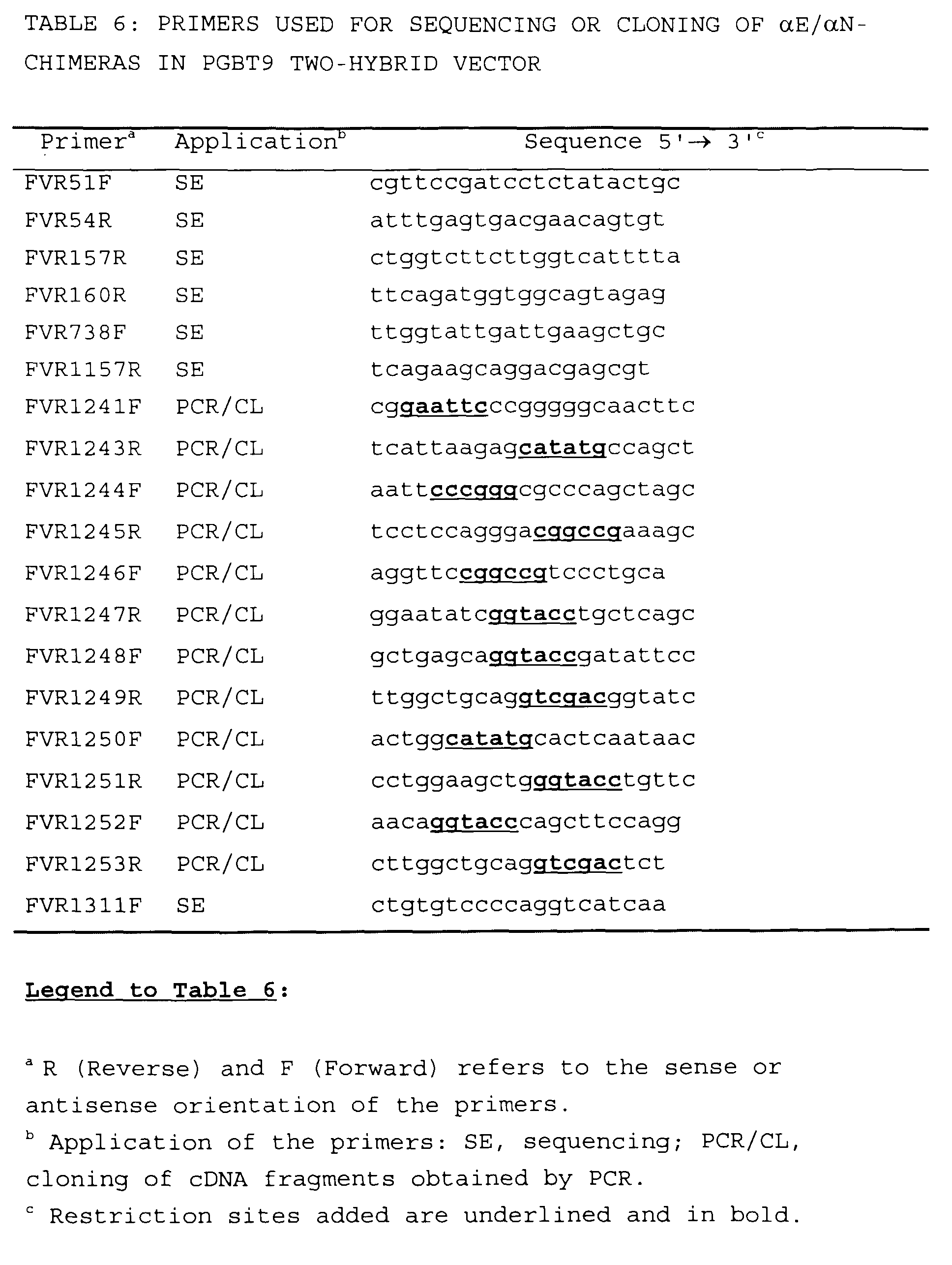

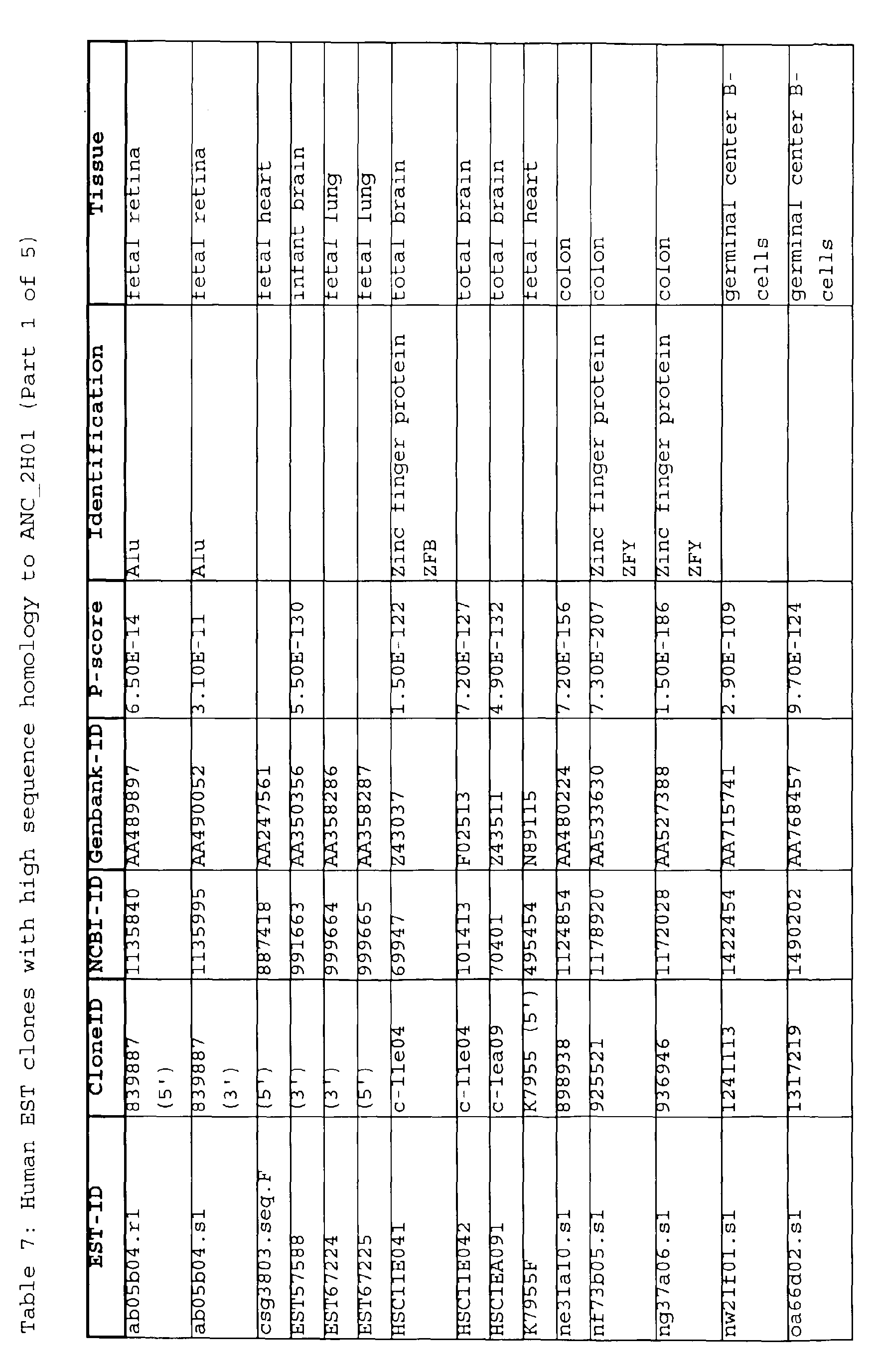

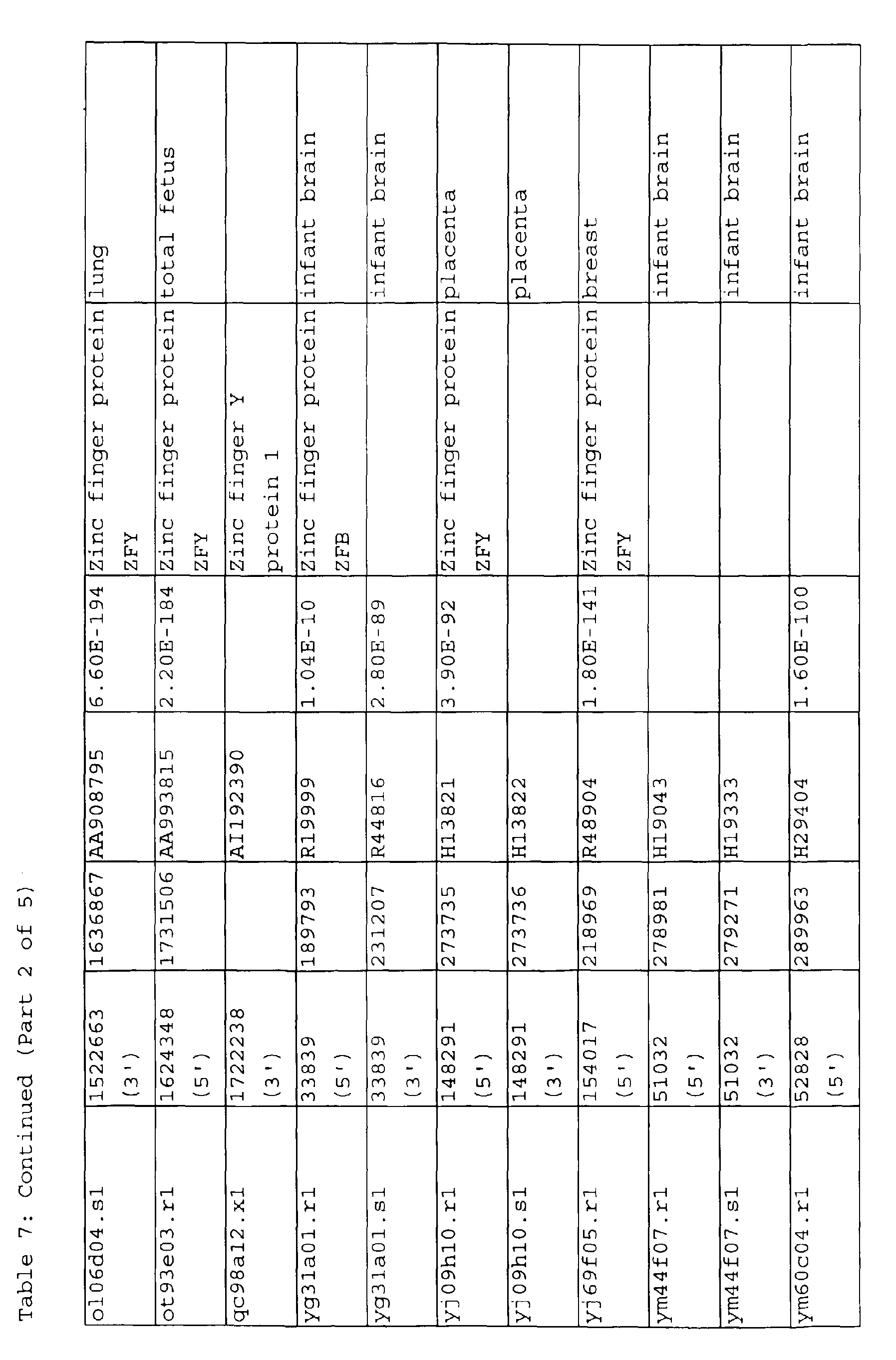

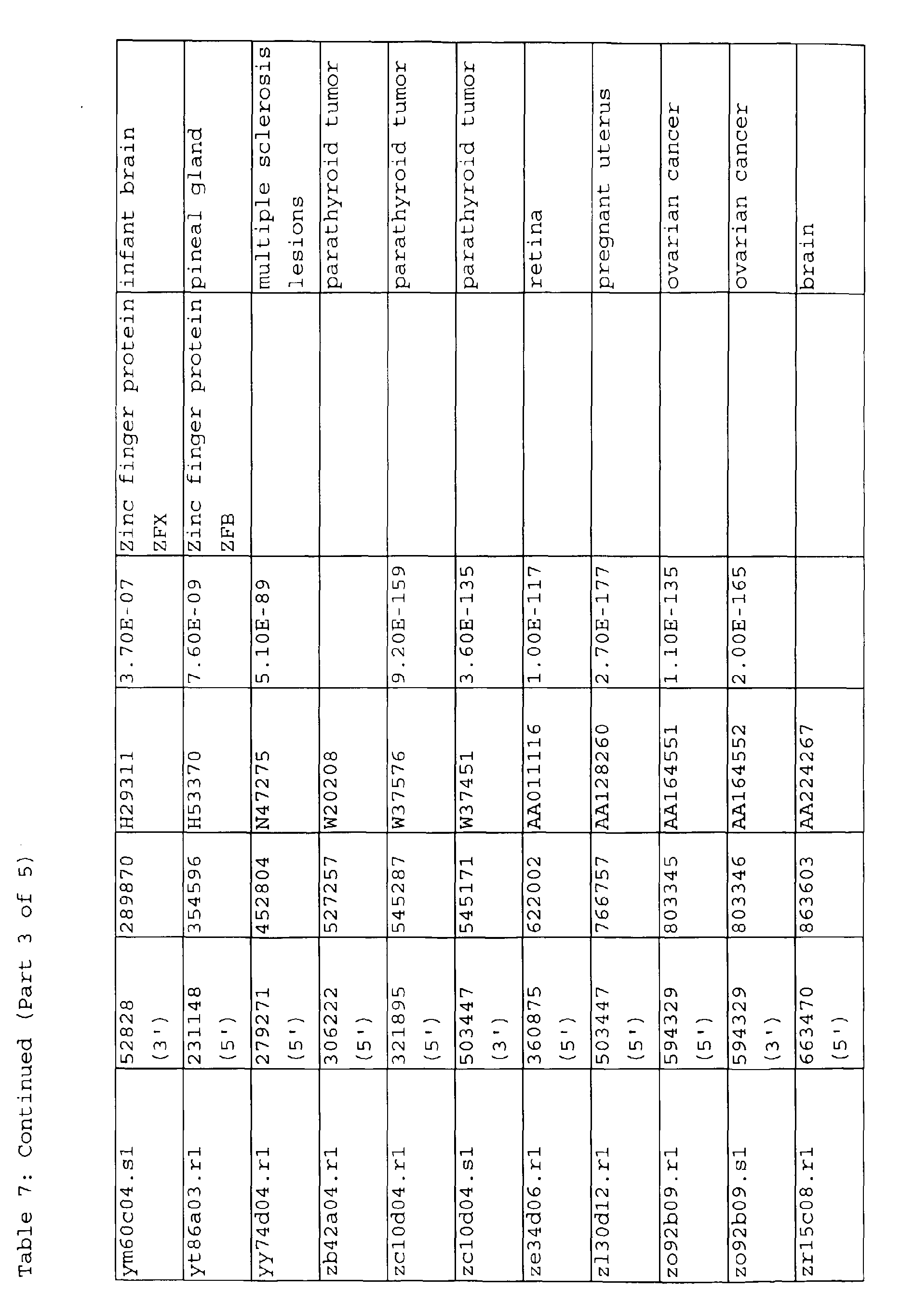

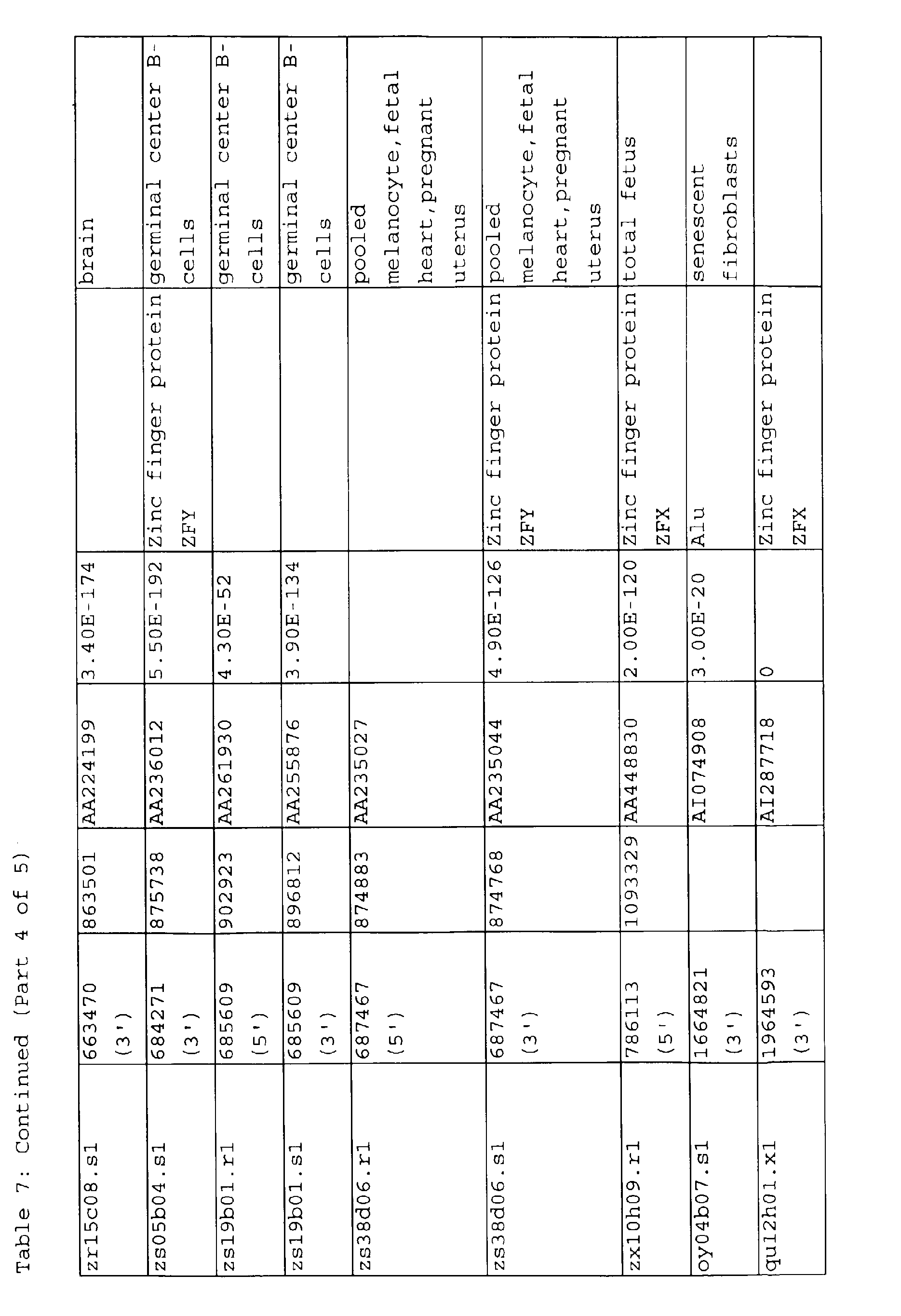

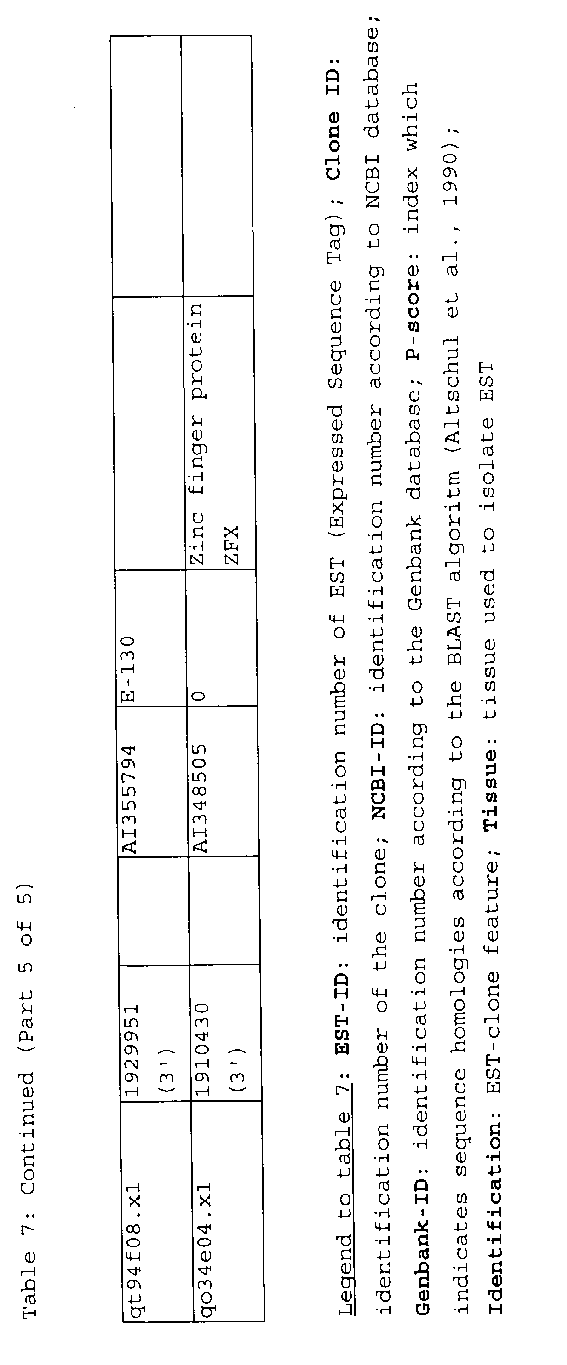

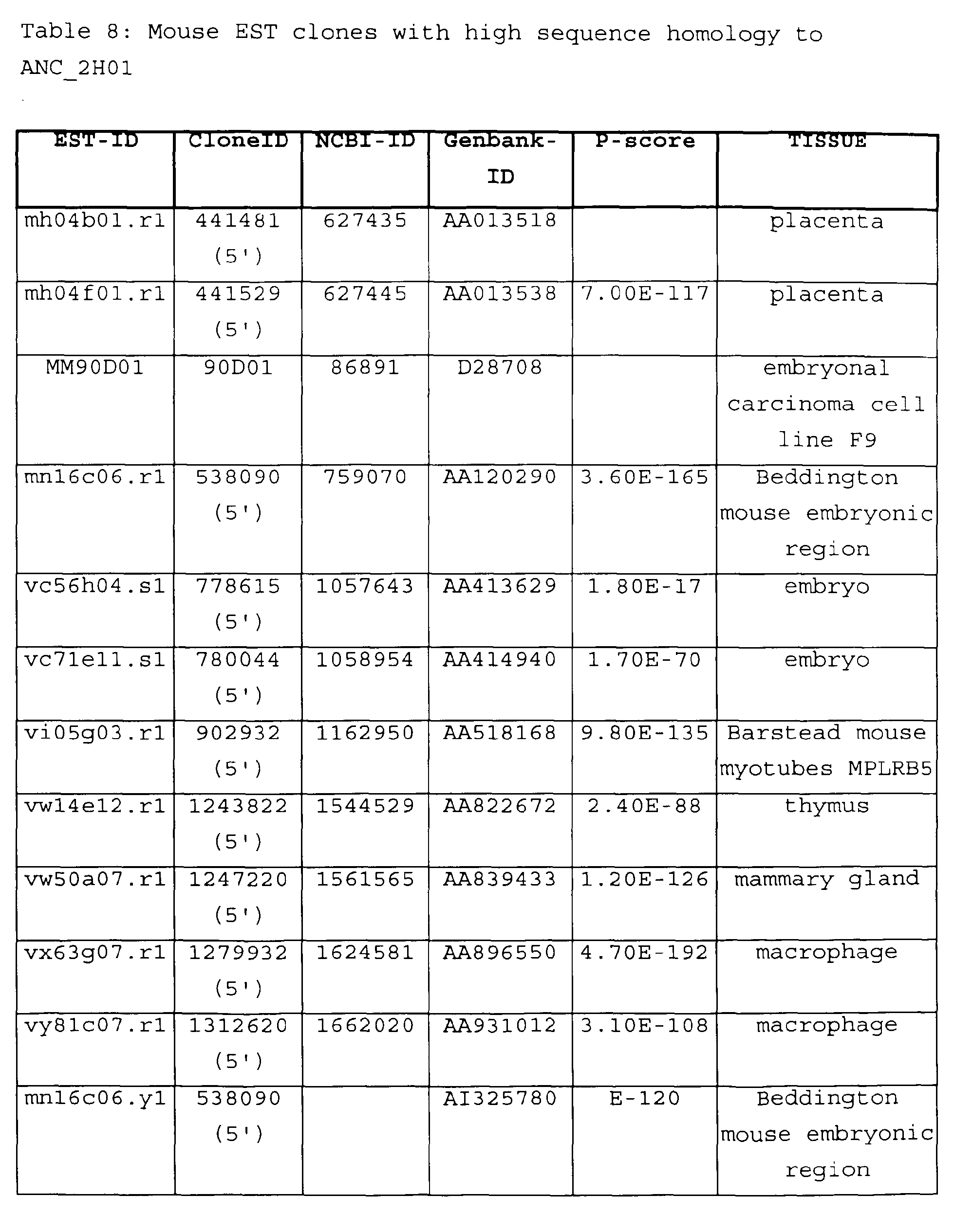

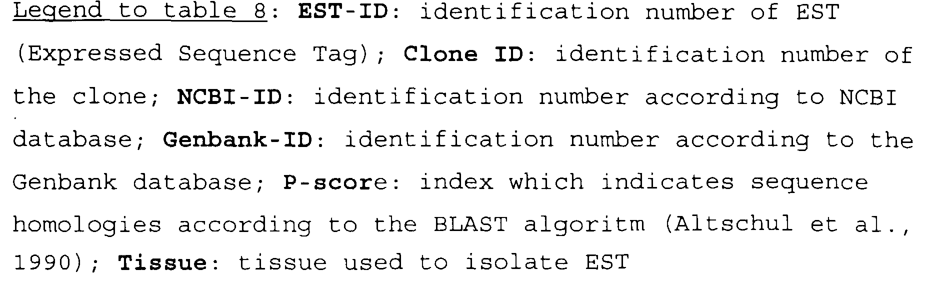

### REFERENCES

Altschul, Stephen, F., Warren, G., Miller, W., Myers, E. W., and Lipman, D. J. (1990). Basic local alignment search tool. J. Mol. Biol. *215*, 403-410.

Bartel, P. L., Chien, C.-T., Sternglanz, R., and Fields, S. (1993). Using the two-hybrid system to detect protein-protein interactions. In Cellular Interactions in Development: A Practical Approach, D. A. Hartley, ed. (Oxford: Oxford University Press), pp. 153-179.

Becker, I., Becker, K. F., Röhrl, M. H., Minkus, G., Schütze, K., and Höfler, H. (1996). Single-cell mutation analysis of tumors from stained histologic slides. Lab. Invest. *75*, 801-807.

Becker, K. F., Atkinson, M. J., Reich, U., Becker, I., Nekarda, H., Siewert, J. R., and Höfler, H. (1994). E-cadherin gene mutations provide clues to diffuse type gastric carcinomas. Cancer Res. *54*, 3845-3852.

Behrens, J., Mareel, M. M., van Roy, F. M., and Birchmeier, W. (1989). Dissecting tumor cell invasion: Epithelial cells acquire invasive properties after the loss of uvomorulin-mediated cell-cell adhesion. J. Cell Biol. *108*, 2435-2447.

Behrens, J., von Kries, J. P., Kühl, M., Bruhn, L., Wedlich, D., Grosschedl, R., and Birchmeier, W. (1996). Functional interaction of beta-catenin with the transcription factor LEF-1. Nature *382*, 638-642.

Berx, G., Cleton-Jansen, A.-M., Nollet, F., de Leeuw, W. J. F., van de Vijver, M. J., Cornelisse, C., and van Roy, F. (1995). E-cadherin is a tumor/invasion suppressor gene mutated in human lobular breast cancers. EMBO J. *14*, 6107-6115.

Berx, G., Cleton-Jansen, A.-M., Strumane, K., de Leeuw, W. J. F., Nollet, F., van Roy, F. M., and Cornelisse, C. (1996). E-cadherin is inactivated in a majority of invasive human lobular breast cancers by truncation mutations throughout its extracellular domain. Oncogene *13*, 1919-1925.

Bonfield, J. K., Smith, K. F., and Staden, R. (1995). A new DNA sequence assembly program. Nucleic Acids Res. *23*, 4992-4999.

Bussemakers, M. J. G., Vandeven, W. J. M., Debruyne, F. M. J., and Schalken, J. A. (1991). Identification of High Mobility Group Protein I(Y) As Potential Progression Marker for Prostate Cancer by Differential Hybridization Analysis. Cancer Res. *51*, 606-611.

Chaganti, S.R., Chen, W. Parsa, N., Offit, K., Louie, D.C., Dalla-Favera, R. and Chaganti, R.S. (1998). Involvement of BCL6 in chromosomal aberrations affecting band 3q27 in B-cell non-Hodgkin lymphoma. Genes Chromosomes and Cancer 23:323-327.

Claverie, J. M., Hardelin, J. P., Legouis, R., Levilliers, J., Bougueleret, L., Mattei, M. G., and Petit, C. (1993). Characterization and chromosomal assignment of a human cDNA encoding a protein related to the murine 102-kDa cadherin-associated protein (alpha-catenin). Genomics *15*, 13-20.

Cowin, P. (1994). Unraveling the cytoplasmic interactions of the cadherin superfamily. Proc. Natl. Acad. Sci. U.S.A. *91*, 10759-10761.

de Leij, L., Postmus, P. E., Buys, C. H. C. M., Elema, J. D., Ramaekers, F., Poppema, S., Brouwer, M., Van Der Veen, A. Y., and Mesander, G. (1985). Characterization of three new variant-type cell lines derived from small cell carcinoma of the lung. Cancer Res. *45*, 6024-6033.

DuBridge, R. B., Tang, P., Hsia, H. C., Leong, P. M., Miller, J. H., and Calos, M. P. (1987). Analysis of mutation in human cells by using an Epstein-Barr virus shuttle system. Mol. Cell. Biol. *7*, 379-387.

Frixen, U. H., Behrens, J., Sachs, M., Eberle, G., Voss, B., Warda, A., Löchner, D., and Birchmeier, W. (1991). E-cadherin-mediated cell-cell adhesion prevents invasiveness of human carcinoma cells. J. Cell Biol. *113,* 173-185.

Georgopoulos, K., Winandy, S., and Avitahl, N. (1997). The role of the Ikaros gene in lymphocyte development and homeostasis. Annu. Rev. Immunol. *15*, 155-176.

Gietz, D., St Jean, A., Woods, R. A., and Schiestl, R. H. (1992). Improved method for high efficiency transformation of intact yeast cells. Nucleic Acids Res. *20*, 1425.

Gorlich, D. and Mattaj, I.W. (1996). Nucleocytoplasmic transport. Science 271:1513-1518.

Graham, F. L., Smiley, J., Russell, W. C., and Nairn, R. (1977). Characteristics of a human cell line transformed by DNA from human adenovirus type 5. J. Gen. Virol. *36*, 59-72.

Hanas, J.S., Bogenhagen, D.F., and Wu, C.W. (1983a). Cooperative model for the binding of Xenopus transcription factor A to the 5S RNA gene. Proc. Natl. Acad. Sci. USA 80:2142-2145.

Hanas, J. S., Hazuda, D. J., Bogenhagen, D. F., Wu, F. Y., and Wu, C. W. (1983b). Xenopus transcription factor A requires zinc for binding to the 5S RNA gene. J. Biol. Chem. *258*, 14120-14125.

He, T. C., Sparks, A. B., Rago, C., Hermeking, H., Zawel, L., da Costa, L. T., Morin, P. J., Vogelstein, B., and Kinzler, K. W. (1998). Identification of c-MYC as a target of the APC pathway. Science *281*, 1509-1512.

Herrenknecht, K., Ozawa, M., Eckerskorn, C., Lottspeich, F., Lenter, M., and Kemler, R. (1991). The uvomorulin-anchorage protein alpha-catenin is a vinculin homologue. Proc. Natl. Acad. Sci. U.S.A. *88*, 9156-9160.

Heusterspreute, M., Ha, T. V., Emery, S., Tournis-Gamble, S., Kennedy, N., and Davison, J. (1985). Vectors with restriction site banks.IV.pJRD184, a 3793-bp plasmid vector with 49 unique restriction sites. Gene *39*, 299-304.

Hirano, S., Kimoto, N., Shimoyama, Y., Hirohashi, S., and Takeichi, M. (1992). Identification of a neural alpha-catenin as a key regulator of cadherin function and multicellular organization. Cell *70*, 293-301.

Huber, O., Korn, R., McLaughlin, J., Ohsugi, M., Herrmann, B. G., and Kemler, R. (1996). Nuclear localization of beta-catenin by interaction with transcription factor LEF-1. Mech. Devel. *59*, 3-10.

Ioannou, P.A. and de Jong, P.J. (1996). Construction of bacterial artificial chromosome libraries using the modified Pl (PAC) system. In: Current protocols in human genetics. Dracopoli, editor. Unit 5.15 Pub. John Wiley and Sons, New York.

James, L.A., Ogilvie, D.J., Yamakawa, K., Nakamura, Y., Stirling, C.J., and Anand, R. (1996). Walking, cloning, and mapping with YACs in 3q27: localization of five ESTs including three membrers of the cystatin gene family and identification of CpG islands. Genomics 32:425-430.

Kalderon, D., Roberts, B. L., Richardson, W. D., and Smith, A. E. (1984). A short amino acid sequence able to specify nuclear location. Cell *39*, 499-509.

Kemler, R. (1992). Classical cadherins. Semin. Cell Biol. *3*, 149-155.

Kievits, T., Dauwerse, J.G., Wiegant, J., Devilee, P., Breuning, M.H., Cornelisse, C.J., Van Ommen, G.J., and Pearson, D.L. (1990). Rapid subchromosomal localization of cosmids by non-radioactive in situ hybridization. Cytogenet. Cell Genet. 53:134-136.

Knudson, A. G. (1985). Hereditary Cancer, Oncogenes, and Antioncogenes. Cancer Res. *45*, 1437-1443.

Korman, N., Eyre, R. W., Klaus-Kovtun, V., and Stanley, J. R. (1989). Demonstration of an adhering junction molecule (plakoglobin) in the autoantigens of pemphigus foliaceous and pemphigus vulgaris. New Engl. J. Med. *321,* 631-635.

Kowalczyk, A. P., Bornslaeger, E. A., Borgwardt, J. E., Palka, H. L., Dhaliwal, A. S., Corcoran, C. M., Denning, M. F., and Green, K. J. (1997). The amino-terminal domain of desmoplakin binds to plakoglobin and clusters desmosomal cadherin-plakoglobin complexes. J. Cell Biol. *139,* 773-784.

Kozak, M. (1996). Interpreting cDNA sequences: Some insights from studies on translation. Mamm. Genome *7*, 563-574.

Kozak, M. (1997). Recognition of AUG and alternative initiator codons is augmented by G in position +4 but is not generally affected by the nucleotides in positions +5 and +6. EMBO Journal. 16:2482-2492.

Laemmli, U. K. (1970). Cleavage of structural proteins during the assembly of the head of bacteriophage T4. Nature *227*, 680-685.

Love, J. J., Li, X., Case, D. A., Giese, K., Grosschedl, R., and Wright, P. E. (1995). Structural basis for DNA bending by the architectural transcription factor LEF-1. Nature *376*, 791-795.

Miller, J., McLachlan, A. D., and Klug, A. (1985). Repetitive zinc-binding domains in the protein transcription factor IIIA from Xenopus oocytes. EMBO J. *4*, 1609-1614.

Mizushima, S., and Nagata, S. (1990). pEF-BOS: a powerful mammalian expression vector. Nucleic Acids Res. *18*, 5322.

Morgan, B., Sun, L., Avitahl, N., Andrikopoulos, K., Ikeda, T., Gonzales, E., Wu, P., Neben, S., and Georgopoulos, K. (1997). Aiolos, a lymphoid restricted transcription factor that interacts with Ikaros to regulate lymphocyte differentiation. EMBO J. *16*, 2004-2013.

Motoyama, T., and Watanabe, H. (1983). Carcinoembryonic antigen production in human gastric cancer cell lines in vitro and in nude mice. Gann *74(5)*, 679-686.

Nagafuchi, A., Takeichi, M., and Tsukita, S. (1991). The 102 kd cadherin-associated protein: Similarity to vinculin and posttranscriptional regulation of expression. Cell *65*, 849-857.

Oda, H., Uemura, T., Shiomi, K., Nagafuchi, A., Tsukita, S., and Takeichi, M. (1993). Identification of a Drosophila homologue of alpha-catenin and its association with the armadillo protein. J. Cell Biol. *121*, 1133-1140.

Ozawa, M., Ringwald, M., and Kemler, R. (1990). Uvomorulin-catenin complex formation is regulated by a specific domain in the cytoplasmic region of the cell adhesion molecule. Proc. Natl. Acad. Sci. U.S.A. *87*, 4246-4250.

Pavletich, N. P., and Pabo, C. O. (1991). Zinc Finger-DNA Recognition - Crystal Structure of a Zif268-DNA Complex at 2.1-A. Science *252*, 809-817.

Pavletich, N. P., and Pabo, C. O. (1993). Crystal structure of a 5-finger GLI-DNA complex - new perspectives on zinc fingers. Science *261*, 1701-1707.

Peifer, M. (1993). Cancer, catenins, and cuticle pattern - a complex connection. Science *262*, 1667-1668.

Peifer, M. (1997). Cancer - beta-catenin as oncogene: The smoking gun. Science *275*, 1752-1753.

Rimm, D. L., Kebriaei, P., and Morrow, J. S. (1994). Molecular cloning reveals alternative splice forms of human alpha(E)-catenin. Biochem. Biophys. Res. Commun. *203*, 1691-1699.

Sanger, F., Nicklen, S., and Coulson, A. (1981). DNA sequencing with chain-terminating inhibitors. Proc. Natl. Acad. Sci. U.S.A. *74*, 5463.

Senoo, M. Seki, N., Ohira, M., Sugano, S., Watanabe, M., Tachibana, M., Tanaka, T., Shinkai, Y., Kato, H. (1998). A second p53-related protein, p73L, with high homology to p73. Biochem. Biophys. Res. Commun. 248:603-607.

Shibuya, Y, Yasuda, H., Tomatsuri, M., Mizoguchi, A., Takeichi, M., Shimada, K., and Ide, C. (1996). Alpha N-catenin expression in the normal and regenerating chick sciatic nerve. J. of Neurocytology. 25:615-624.

Su, L. K., Vogelstein, B., and Kinzler, K. W. (1993). Association of the APC tumor suppressor protein with catenins. Science *262*, 1734-1737.

Sun, L., Liu, A., and Georgopoulos, K. (1996). Zinc finger-mediated protein interactions modulate ikaros activity, a molecular control of lymphocyte development. EMBO J. *15*, 5358-5369.

Suzuki, S. T. (1996). Structural and functional diversity of cadherin superfamily: Are new members of cadherin superfamily involved in signal transduction pathway? J. Cell. Biochem. *61*, 531-542.

Takeichi, M. (1991). Cadherin cell adhesion receptors as a morphogenetic regulator. Science *251*, 1451-1455.

Tetsu, O., and McCormick, F. (1999). Beta-catenin regulates expression of cyclin D1 in colon carcinoma cells. Nature *398*, 422-426.

Uchida, N., Shimamura, K., Miyatani, S., Copeland, N. G., Gilbert, D. J., Jenkins, N. A., and Takeichi, M. (1994). Mouse alpha-N-catenin: Two isoforms, specific expression in the nervous system, and chromosomal localization of the gene. Dev. Biol. *163*, 75-85.

Vleminckx, K., Vakaet Jr, L., Mareel, M., Fiers, W., and Van Roy, F. (1991). Genetic manipulation of E-cadherin expression by epithelial tumor cells reveals an invasion suppressor role. Cell *66*, 107-119.

Wigler, M., Pellicer, A., Silverstein, S., and Axel, R. (1978). Biochemical transfer of single-copy eucaryotic genes using total cellular DNA as donor. Cell *14*, 725-731.

Wright, W. E., Binder, M., and Funk, W. (1991). Cyclic amplification and selection of targets (CASTing) for the myogenin consensus binding site. Mol. Cell. Biol. *11*, 4104-4110.

Yang, A., Kaghad M., Wang Y., Gillet E., Fleming M.D., Dotsch V., Andrews N.C., Caput D. and McKeon, F. (1998). P63, a p53 homolog at 3q27-29, encodes multiple products with transactivating death-inducing and dominant-negative activities. Moll. Cell. 2:305-316.

## Claims

1. An isolated and/or recombinant nucleic acid or a functional fragment, homologue or derivative thereof, corresponding to a catenin-binding protein with function in signal transduction or gene regulatory pathways.

2. An isolated and/or recombinant nucleic acid or a functional fragment, homologue or derivative thereof, according to claim 1 wherein said molecule is corresponding to a zinc finger gene with a nucleic acid sequence as shown in figure 1 and encoding a zinc finger protein, or fragment thereof, capable of complexing with catenin.

3. A nucleic acid according to claim 1 or 2 wherein said protein is capable of nuclear translocation of a catenin.

4. A nucleic acid according to claim 1,2 or 3 wherein said catenin comprises αN-catenin.

5. A nucleic acid according to anyone of claims 1 to 4 which is a cDNA molecule.

6. An expression vector comprising a nucleic acid according to anyone of claims 1 to 5.

7. A cell comprising a nucleic acid according to anyone of claims 1 to 5 or a vector according to claim 6.

8. A cell according to claim 7 wherein said cell is a yeast cell or a mammalian cell.

9. A cell capable of expressing a zinc finger protein or fragment thereof, said protein capable of complexing with a catenin.

10. A cell according to claim 9 wherein said catenin comprises αN-catenin.

11. An animal comprising a cell according to anyone of claims 7, 9 or 10.

12. A zinc finger protein or derivative or fragment thereof, said protein capable of complexing with a catenin, preferably with αN-catenin.

13. A protein or derivative or fragment thereof according to claim 12 encoded by a nucleic acid according to anyone of claims 1 to 5.

14. An antibody specifically directed against a protein or derivative or fragment thereof according to claim 13.

15. A method for identifying a candidate drug comprising use of a cell according to any of claims 7 to 10 or an animal according to claim 11 or a protein according to claim 12 or 13 or an antibody according to claim 14.

16. A method according to claim 15 wherein said drug is for use in a cancer patient or in a patient with a neurological disorder.

17. A method for diagnosing, prognosing and/or treating cancer or neurological disorders, comprising use of a nucleic acid according to anyone of claims 1 to 5, a cell according to any of claims 7 to 10 or an animal according to claim 11 or a protein according to claim 12 or 13 or an antibody according to claim 14.


### Annex to the application documents - subsequently filed sequences listing

SEQUENCE LISTING

<110> VLAAMS INTERUNIVERSITAIR INSTITUUT VOOR BIOTECHNOL

<120> Novel cDNAs encoding catenin-binding proteins with
      function in signalling and/or gene regulation

<130> V1/001-041/EP

<140> ep 99201543.8
<141> 1999-05-17

<160> 83

<170> PatentIn Ver. 2.1

<210> 1
<211> 20
<212> DNA
<213> Artificial Sequence

<220>
<223> Description of Artificial Sequence: primer FVR274F

<400> 1
actgctgaag atgttccaat                                              20


<210> 2
<211> 20
<212> DNA
<213> Artificial Sequence

<220>
<223> Description of Artificial Sequence: primer FVR291F

<400> 2
aggatcccta catttgtaaa                                              20


<210> 3
<211> 20
<212> DNA
<213> Artificial Sequence

<220>
<223> Description of Artificial Sequence: primer FVR293F

<400> 3
ttgtgatgac tgtgggaaag                                                    20


<210> 4
<211> 20
<212> DNA
<213> Artificial Sequence

<220>
<223> Description of Artificial Sequence: primer FVR308R

<400> 4
cctgggcaac atagcgagat                                                    20


<210> 5
<211> 20
<212> DNA
<213> Artificial Sequence

<220>
<223> Description of Artificial Sequence: primer FVR309R

<400> 5
ggactctcct cttgggtttg                                                    20


<210> 6
<211> 20
<212> DNA
<213> Artificial Sequence

<220>
<223> Description of Artificial Sequence: primer FVR310R

<400> 6
tctgtttctg gccttgattc                                                    20


<210> 7
<211> 27
<212> DNA
<213> Artificial Sequence

<220>
<223> Description of Artificial Sequence: primer FVR345F

<400> 7
gcactatggc cagaaacaga aatcaga                                          27


<210> 8
<211> 27
<212> DNA
<213> Artificial Sequence

<220>
<223> Description of Artificial Sequence: primer FVR346R

<400> 8
ggaattcctg ggcagtcaca ttcaaag                                          27


<210> 9
<211> 27
<212> DNA
<213> Artificial Sequence

<220>
<223> Description of Artificial Sequence: primer FVR347R

<400> 9
ggaattccat atgctgcttt aagtcag                                          27


<210> 10
<211> 20
<212> DNA
<213> Artificial Sequence

<220>
<223> Description of Artificial Sequence: primer FVR359R

<400> 10
gcggttcttc atcagtttgg                                                  20


<210> 11
<211> 20
<212> DNA
<213> Artificial Sequence

<220>
<223> Description of Artificial Sequence: primer FVR360R

<400> 11
ctcttgggtt tgctggttga                                                    20

<210> 12
<211> 20
<212> DNA
<213> Artificial Sequence

<220>
<223> Description of Artificial Sequence: primer FVR421R

<400> 12
aacgagaagg gtgtagagtc                                                    20

<210> 13
<211> 20
<212> DNA
<213> Artificial Sequence

<220>
<223> Description of Artificial Sequence: primer FVR462R

<400> 13
tcctgtaata gtggcttcct                                                    20

<210> 14
<211> 20
<212> DNA
<213> Artificial Sequence

<220>
<223> Description of Artificial Sequence: primer FVR463F

<400> 14
tgatgactgt gggaaaggct                                                    20

<210> 15
<211> 20
<212> DNA
<213> Artificial Sequence

<220>
<223> Description of Artificial Sequence: primer FVR464R

<400> 15
ggaggcggag gttgtggtga                                                    20


<210> 16
<211> 20
<212> DNA
<213> Artificial Sequence

<220>
<223> Description of Artificial Sequence: primer FVR465R

<400> 16
cctccctcat cccatcttaa                                                    20


<210> 17
<211> 20
<212> DNA
<213> Artificial Sequence

<220>
<223> Description of Artificial Sequence: primer FVR508F

<400> 17
ccaaagcctc cagcctgaga                                                    20


<210> 18
<211> 20
<212> DNA
<213> Artificial Sequence

<220>
<223> Description of Artificial Sequence: primer FVR509R

<400> 18
aggccgaggc gaacagatca                                                    20


<210> 19
<211> 20
<212> DNA
<213> Artificial Sequence

<220>
<223> Description of Artificial Sequence: primer FVR510F

<400> 19
gctgggatta caggcgtgaa                                                    20


<210> 20
<211> 20
<212> DNA
<213> Artificial Sequence

<220>
<223> Description of Artificial Sequence: primer FVR511F

<400> 20
tcaagcagtg aactctacct                                                    20


<210> 21
<211> 20
<212> DNA
<213> Artificial Sequence

<220>
<223> Description of Artificial Sequence: primer FVR512R

<400> 21
aactctatta atttacatgc                                                    20


<210> 22
<211> 20
<212> DNA
<213> Artificial Sequence

<220>
<223> Description of Artificial Sequence: primer FVR513F

<400> 22
aacaatggtg aacatggaca                                                    20


<210> 23
<211> 20
<212> DNA
<213> Artificial Sequence

<220>
<223> Description of Artificial Sequence: primer FVR514R

<400> 23
tcactacatt tatgaggcaa                                                    20

<210> 24
<211> 20
<212> DNA
<213> Artificial Sequence

<220>
<223> Description of Artificial Sequence: primer FVR515F

<400> 24
ccatgagaca acttgattat                                                    20

<210> 25
<211> 20
<212> DNA
<213> Artificial Sequence

<220>
<223> Description of Artificial Sequence: primer FVR516F

<400> 25
gcgtgatagt ttgtagttta                                                    20

<210> 26
<211> 20
<212> DNA
<213> Artificial Sequence

<220>
<223> Description of Artificial Sequence: primer FVR517F

<400> 26
cgaccagaac taaaatgcaa                                                    20

<210> 27
<211> 20
<212> DNA
<213> Artificial Sequence

<220>
<223> Description of Artificial Sequence: primer FVR518F

<400> 27
agatggtctc gttctgttgt                                                    20


<210> 28
<211> 20
<212> DNA
<213> Artificial Sequence

<220>
<223> Description of Artificial Sequence: primer FVR519F

<400> 28
tgcctgactc ttgcccaaat                                                    20


<210> 29
<211> 37
<212> DNA
<213> Artificial Sequence

<220>
<223> Description of Artificial Sequence: primer FVR660F

<400> 29
atcgtcagcg acataggtca atggaatttt ctctgat                                 37


<210> 30
<211> 37
<212> DNA
<213> Artificial Sequence

<220>
<223> Description of Artificial Sequence: primer FVR661R

<400> 30
ataagaatgc ggccgctgtt gtctcatgga ctggaag                                 37


<210> 31
<211> 36
<212> DNA
<213> Artificial Sequence

<220>
<223> Description of Artificial Sequence: primer FVR662F

<400> 31
ataagaatgc ggccgctatg aatgagtatc ctaaaa                                36

<210> 32
<211> 35
<212> DNA
<213> Artificial Sequence

<220>
<223> Description of Artificial Sequence: primer FVR663R

<400> 32
cggatacagc atagcgtaga aaaggcagtg tggtc                                 35

<210> 33
<211> 29
<212> DNA
<213> Artificial Sequence

<220>
<223> Description of Artificial Sequence: primer
      FVR1043F

<400> 33
cgtggatccg aaaagatatg aatgagtat                                        29

<210> 34
<211> 29
<212> DNA
<213> Artificial Sequence

<220>
<223> Description of Artificial Sequence: primer
      FVR1044F

<400> 34
cctctcgagc aaagttcaca tttatagag                                        29

<210> 35
<211> 27
<212> DNA
<213> Artificial Sequence

<220>

<223> Description of Artificial Sequence: primer
      FVR1045R

<400> 35
ggaattcgcc tctataaatg tgaactt                                          27


<210> 36
<211> 29
<212> DNA
<213> Artificial Sequence

<220>
<223> Description of Artificial Sequence: primer
      FVR1046R

<400> 36
ccgctcgaga agttaaagag aataatcaa                                        29


<210> 37
<211> 28
<212> DNA
<213> Artificial Sequence

<220>
<223> Description of Artificial Sequence: primer
      FVR1237F

<400> 37
ggaattcctg aatgagtatc ctaaaaaa                                         28


<210> 38
<211> 28
<212> DNA
<213> Artificial Sequence

<220>
<223> Description of Artificial Sequence: primer
      FVR1238R

<400> 38
atgcatgctg tagaaaaggc agtgtggt                                         28


<210> 39
<211> 30

<212> DNA
<213> Artificial Sequence

<220>
<223> Description of Artificial Sequence: primer
      FVR1240F

<400> 39
cgtcgcggcc ctgcagatgg attcaatgga                                        30


<210> 40
<211> 30
<212> DNA
<213> Artificial Sequence

<220>
<223> Description of Artificial Sequence: primer
      FVR1242R

<400> 40
tccccccggg gggatgaatt tattatttta                                        30


<210> 41
<211> 31
<212> DNA
<213> Artificial Sequence

<220>
<223> Description of Artificial Sequence: primer
      FVR1411F

<400> 41
tcccccgggt atgaatgagt atcctaaaaa a                                      31


<210> 42
<211> 30
<212> DNA
<213> Artificial Sequence

<220>
<223> Description of Artificial Sequence: primer
      FVR1412R

<400> 42
aaaagtcgac ggccactgct attagctctc                                        30

<210> 43
<211> 28
<212> DNA
<213> Artificial Sequence

<220>
<223> Description of Artificial Sequence: primer
      FVR1413F

<400> 43
ggaattcttc tataaatgtg aactttgt                                        28


<210> 44
<211> 30
<212> DNA
<213> Artificial Sequence

<220>
<223> Description of Artificial Sequence: primer
      FVR1414R

<400> 44
aaaagtcgac aagttaaaga gaataatcaa                                      30


<210> 45
<211> 20
<212> DNA
<213> Artificial Sequence

<220>
<223> Description of Artificial Sequence: primer FVR174F

<400> 45
accactacaa tggatgatgt                                                 20


<210> 46
<211> 20
<212> DNA
<213> Artificial Sequence

<220>
<223> Description of Artificial Sequence: primer FVR175F

<400> 46
atcatcggaa gagagtagta                                                    20


<210> 47
<211> 20
<212> DNA
<213> Artificial Sequence

<220>
<223> Description of Artificial Sequence: primer FVR192R

<400> 47
taaaagaagg caaaacgatg                                                    20


<210> 48
<211> 20
<212> DNA
<213> Artificial Sequence

<220>
<223> Description of Artificial Sequence: primer FVR217R

<400> 48
aaaatcataa atcataagaa                                                    20


<210> 49
<211> 34
<212> DNA
<213> Artificial Sequence

<220>
<223> Description of Artificial Sequence: primer FVR239F

<220>
<221> misc_feature
<222> (22)..(23)
<223> modified-base: inosine

<220>
<221> misc_feature
<222> (27)..(28)
<223> modified-base: inosine

<220>
<221> misc_feature

<222> (32)..(33)
<223> modified-base: inosine

<400> 49
gaattcgtcg actagtacgg gnngggnngg gnng                                34


<210> 50
<211> 18
<212> DNA
<213> Artificial Sequence

<220>
<223> Description of Artificial Sequence: primer FVR240F

<400> 50
gaattcgtcg actagtac                                                  18


<210> 51
<211> 30
<212> DNA
<213> Artificial Sequence

<220>
<223> Description of Artificial Sequence: primer FVR137F

<400> 51
accccccggg ggcaacttca cctatcattc                                    30


<210> 52
<211> 30
<212> DNA
<213> Artificial Sequence

<220>
<223> Description of Artificial Sequence: primer 138R

<400> 52
gccgccgcct tccttttcat ttccgctctt                                     30


<210> 53
<211> 24
<212> DNA
<213> Artificial Sequence

<220>
<223> Description of Artificial Sequence: primer FVR283

<400> 53
cgccagggtt ttcccagtca cgac                                          24


<210> 54
<211> 22
<212> DNA
<213> Artificial Sequence

<220>
<223> Description of Artificial Sequence: primer FVR284

<400> 54
tcacacagga aacagctatg ac                                            22


<210> 55
<211> 21
<212> DNA
<213> Artificial Sequence

<220>
<223> Description of Artificial Sequence: primer FVR51F

<400> 55
cgttccgatc ctctatactg c                                             21


<210> 56
<211> 20
<212> DNA
<213> Artificial Sequence

<220>
<223> Description of Artificial Sequence: primer FVR54R

<400> 56
atttgagtga cgaacagtgt                                               20


<210> 57
<211> 21
<212> DNA
<213> Artificial Sequence

<220>
<223> Description of Artificial Sequence: primer FVR157R

<400> 57
ctggtcttct tggtcatttt a                                                21


<210> 58
<211> 20
<212> DNA
<213> Artificial Sequence

<220>
<223> Description of Artificial Sequence: primer FVR160R

<400> 58
ttcagatggt ggcagtagag                                                  20


<210> 59
<211> 20
<212> DNA
<213> Artificial Sequence

<220>
<223> Description of Artificial Sequence: primer FVR738F

<400> 59
ttggtattga ttgaagctgc                                                  20


<210> 60
<211> 19
<212> DNA
<213> Artificial Sequence

<220>
<223> Description of Artificial Sequence: primer
      FVR1157R

<400> 60
tcagaagcag gacgagcgt                                                   19


<210> 61
<211> 22
<212> DNA
<213> Artificial Sequence

<220>
<223> Description of Artificial Sequence: primer
      FVR1241F

<400> 61
cggaattccc gggggcaact tc                                                22


<210> 62
<211> 22
<212> DNA
<213> Artificial Sequence

<220>
<223> Description of Artificial Sequence: primer
      FVR1243R

<400> 62
tcattaagag catatgccag ct                                                22


<210> 63
<211> 22
<212> DNA
<213> Artificial Sequence

<220>
<223> Description of Artificial Sequence: primer
      FVR1244F

<400> 63
aattcccggg cgcccagcta gc                                                22


<210> 64
<211> 22
<212> DNA
<213> Artificial Sequence

<220>
<223> Description of Artificial Sequence: primer
      FVR1245R

<400> 64
tcctccaggg acggccgaaa gc                                                22

<210> 65
<211> 20
<212> DNA
<213> Artificial Sequence

<220>
<223> Description of Artificial Sequence: primer
      FVR1246F

<400> 65
aggttccggc cgtccctgca                                                20


<210> 66
<211> 22
<212> DNA
<213> Artificial Sequence

<220>
<223> Description of Artificial Sequence: primer
      FVR1247R

<400> 66
ggaatatcgg tacctgctca gc                                             22


<210> 67
<211> 22
<212> DNA
<213> Artificial Sequence

<220>
<223> Description of Artificial Sequence: primer
      FVR1248F

<400> 67
gctgagcagg taccgatatt cc                                             22


<210> 68
<211> 22
<212> DNA
<213> Artificial Sequence

<220>
<223> Description of Artificial Sequence: primer
      FVR1249R

<400> 68
ttggctgcag gtcgacggta tc                                                22

<210> 69
<211> 22
<212> DNA
<213> Artificial Sequence

<220>
<223> Description of Artificial Sequence: primer
      FVR1250F

<400> 69
actggcatat gcactcaata ac                                                22

<210> 70
<211> 22
<212> DNA
<213> Artificial Sequence

<220>
<223> Description of Artificial Sequence: primer
      FVR1251R

<400> 70
cctggaagct gggtacctgt tc                                                22

<210> 71
<211> 21
<212> DNA
<213> Artificial Sequence

<220>
<223> Description of Artificial Sequence: primer FR1252F

<400> 71
aacaggtacc cagcttccag g                                                 21

<210> 72
<211> 20
<212> DNA
<213> Artificial Sequence

<220>

<223> Description of Artificial Sequence: primer
      FVR1253R

<400> 72
cttggctgca ggtcgactct                                                20


<210> 73
<211> 20
<212> DNA
<213> Artificial Sequence

<220>
<223> Description of Artificial Sequence: primer
      FVR1311F

<400> 73
ctgtgtcccc aggtcatcaa                                                20


<210> 74
<211> 3013
<212> DNA
<213> Homo sapiens

<400> 74
caacgagttg tagccgcgga gagcaggcgt cgatgctggc gcccaaagcc tccagcctga 60
gagtcgcctc actcgtgtga cgacagagtt tcactcctgt tacccaggct ggaggacagt 120
gatgtgatct cgggtcacca caacctccgc ctcccggatt caggcgattc tcatgcctca 180
gcctcccgag tagctcagat tacaggcatg tgccaccacg cccggctaat tttgtatttt 240
cagtcgagac ggggtttccc catgttggtc aggctggtct tgaattcccg acctcaggtg 300
atctgttcgc ctcggcctcc caaagtgctg ggattacagg cgtgaaccac tgcacccggc 360
gaggcatttt ttactgtcta cagaaactta ttgtaattca tttttcctca ctccagtagt 420
aagaattata ccaaattgaa aagatatgaa tgagtatcct aaaaaaagaa aaaggaagac 480
tctacaccct tctcgttatt cagattcctc tggaataagc agaattgcag atggattcaa 540
tggaattttc tctgatcatt gttacagtgt ctgttctatg agacagccag atttaaaata 600
ttttgacaac aaagatgatg attctgatac cgagacgtca aatgacttgc caaaatttgc 660
agatggaatc aaggccagaa acagaaatca gaactacctg gttcccagtc ctgtacttag 720
aattctagac cacactgcct tttctacaga aaaatctgct gatattgtaa tttgtgatga 780
agagtgtgac tcacctgaat cagtcaacca gcaaacccaa gaggagagtc ctatagaagt 840
tcacactgct gaagatgttc caattgctgt agaagtgcat gcgatttctg aggattatga 900
tatagagaca gaaaacaatt cctctgagag tctccaagac caaactgatg aagaaccgcc 960
agctaaactt tgtaaaattc ttgacaagag ccaagctttg aatgtgactg cccagcagaa 1020
atggccttta ctgagagcta atagcagtgg cctctataaa tgtgaacttt gtgagtttaa 1080
cagcaaatat ttttctgact taaagcagca tatgatcctg aagcataaac gtactgattc 1140
aaatgtgtgt cgagtatgca aggaaagttt ctctaccaat atgcttctga tagaacatgc 1200
caaactgcat gaagaggatc cctacatttg taaatactgt gattataaga cagtaatttt 1260
tgagaacctc agccagcaca ttgcagacac ccattttagt gatcacctct attggtgtga 1320

acagtgtgat gtacagttct cctcaagcag tgaactctac ctacatttcc aggagcacag 1380
ctgtgatgaa cagtacttgt gtcagttctg tgaacatgaa actaatgatc cagaagactt 1440
gcatagccat gtggtaaatg agcatgcatg taaattaata gagttaagtg ataagtataa 1500
caatggtgaa catggacaat atagcctctt aagcaaaatt acctttgaca aatgtaaaaa 1560
cttctttgta tgtcaagtat gtggttttcg gagtagactt cacacaaatg ttaacaggca 1620
tgttgctatt gaacatacaa aaatttttcc tcatgtttgt gatgactgtg ggaaaggctt 1680
ttcaagtatg ctagaatatt gcaagcattt aaattcacat ttatctgaag ggatttattt 1740
atgtcaatat tgtgaatatt caacaggaca aattgaagat cttaaaattc atctagattt 1800
caagcattca gctgacttgc ctcataaatg tagtgactgc ttgatgaggt ttggaaatga 1860
aagggaatta ataagtcacc ttccagtcca tgagacaact tgattattct ctttaactta 1920
cagaatgtta gtttaaaata ataaattcat cctttttttg gagatgatta aatggatgat 1980
tgtaaacaca acttatgaaa tctgccttta acaagtaact tttttaaatt ataaaatttt 2040
attggcattg ctccattttc tgtatataaa tatatcttta atgtggtatt ttcaattgcg 2100
tgatagtttg tagtttcaac cactcttggt gactgtcatc ctgtttcttc catattctct 2160
gatttcatga attgaaaaga aacaaatgta ttgaagaagt gagctacagt tttccttcct 2220
taaccatggg tgctagtaac tttttaaaac tcaagacaag attagttttt tatgtgtgaa 2280
gtcattaaat tattacacga ccagaactaa aatgcaatat acagttaagt ccacggatac 2340
tcccattaat gagaaataac actaggaagc cactattaca ggaagaaaag atttggtttt 2400
catggcagtc tgttttttta aaaaaaaatt tttgagccac tatctattgt tgaatatttt 2460
aagatgggat gagggaggaa ctaataaggg cttacacaat aaaaaataac tatatcataa 2520
ctcattcata acttgatgtt tcattttctg ttgaggaacc ataaattcat tcacagactt 2580
aatatttttt tcttagagat ggtctcgttc tgttgtccag gatggagtgc agtggttgat 2640
catagctccc gggccgtagt ctcccaggct cgagcaatcc tcccacttca ccctcctgca 2700
tagctaggac tacaggcatg tggcaccatg cccgctaagt ttttaaattt cttgtagaga 2760
tgagatctcg ctatgttgcc caggccagtc tcaaactcct ggactcaagc aatcctccca 2820
ccttggcctt ccaaatcact gggattatag gcatgagcca ttatgcctga ctcttgccca 2880
aatttctgat gtcaaattgt tcattgacag aaaacccact gaagtattta aagttaggaa 2940
gatctgggag ataggggttg ctggcatgaa aatgtataac ttacaacatt tattaataaa 3000
atgataaatt agc 3013

<210> 75
<211> 24
<212> PRT
<213> Artificial Sequence

<220>
<223> Description of Artificial Sequence: zinc finger
motif: ZF1

<400> 75
Tyr Lys Cys Glu Leu Cys Glu Phe Asn Ser Lys Tyr Phe Ser Asp Leu
1               5                   10                  15

Lys Gln His Met Ile Leu Lys His
            20

<210> 76
<211> 23
<212> PRT
<213> Artificial Sequence

<220>
<223> Description of Artificial Sequence: zinc finger
      motif: ZF2

<400> 76
Asn Val Cys Arg Val Cys Lys Glu Ser Phe Ser Thr Asn Met Leu Leu
  1               5                  10                  15

Ile Glu His Ala Lys Leu His
             20

<210> 77
<211> 24
<212> PRT
<213> Artificial Sequence

<220>
<223> Description of Artificial Sequence: zinc finger
      motif: ZF3

<400> 77
Tyr Ile Cys Lys Tyr Cys Asp Tyr Lys Thr Val Ile Phe Glu Asn Leu
  1               5                  10                  15

Ser Gln His Ile Ala Asp Thr His
             20

<210> 78
<211> 23
<212> PRT
<213> Artificial Sequence

<220>
<223> Description of Artificial Sequence: zinc finger
      motif: ZF4

<400> 78
Tyr Trp Cys Glu Gln Cys Asp Val Gln Phe Ser Ser Ser Ser Glu Leu

1                   5                   10                  15

Tyr Leu His Phe Gln Glu His
                20

<210> 79
<211> 24
<212> PRT
<213> Artificial Sequence

<220>
<223> Description of Artificial Sequence: zinc finger
      motif: ZF5

<400> 79
Tyr Leu Cys Gln Phe Cys Glu His Glu Thr Asn Asp Pro Glu Asp Leu
  1               5                   10                  15

His Ser His Val Val Asn Glu His
                20

<210> 80
<211> 24
<212> PRT
<213> Artificial Sequence

<220>
<223> Description of Artificial Sequence: zinc finger
      motif: ZF6

<400> 80
Phe Val Cys Gln Val Cys Gly Phe Arg Ser Arg Leu His Thr Asn Val
  1               5                   10                  15

Asn Arg His Val Ala Ile Glu His
                20

<210> 81
<211> 23
<212> PRT
<213> Artificial Sequence

<220>
<223> Description of Artificial Sequence: zinc finger
      motif: ZF7

<400> 81
His Val Cys Asp Asp Cys Gly Lys Gly Phe Ser Ser Met Leu Glu Tyr
  1               5                  10                  15

Cys Lys His Leu Asn Ser His
             20

<210> 82
<211> 24
<212> PRT
<213> Artificial Sequence

<220>
<223> Description of Artificial Sequence: zinc finger
      motif: ZF8

<400> 82
Tyr Leu Cys Gln Tyr Cys Glu Tyr Ser Thr Gly Gln Ile Glu Asp Leu
  1               5                  10                  15

Lys Ile His Leu Asp Phe Lys His
             20

<210> 83
<211> 23
<212> PRT
<213> Artificial Sequence

<220>
<223> Description of Artificial Sequence: zinc finger
      motif:  ZF9

<400> 83
His Lys Cys Ser Asp Cys Leu Met Arg Phe Gly Asn Glu Arg Glu Leu
  1               5                  10                  15

Ile Ser His Leu Pro Val His
             20